# EUROPEAN PATENT SPECIFICATION

(11) **EP 3 938 542 B1**
(45) Date of publication and mention of the grant of the patent: **27.08.2025**
(21) Application number: 20708152.2
(22) Date of filing: 11.03.2020
(51) Int. Cl.: C12Q 1/689, C12Q 1/6886, G01N 33/574

(54) **IMPROVED METHOD FOR THE SCREENING, DIAGNOSIS AND/OR MONITORING OF COLORECTAL ADVANCED NEOPLASIA, ADVANCED ADENOMA AND/OR COLORECTAL CANCER**
VERBESSERTES VERFAHREN ZUM SCREENING, DIAGNOSTIZIEREN UND/ODER ÜBERWACHEN VON KOLOREKTALER FORTGESCHRITTENER NEOPLASIE, FORTGESCHRITTENER ADENOM UND/ODER KOLOREKTALKREBS
PROCÉDÉ AMÉLIORÉ POUR LE CRIBLAGE, LE DIAGNOSTIC ET/OU LA SURVEILLANCE D'UNE NÉOPLASIE COLORECTALE AVANCÉE, D'UN ADÉNOME AVANCÉ ET/OU D'UN CANCER COLORECTAL

(30) Priority: 13.03.2019 EP 19382186; 26.09.2019 EP 19382833
(43) Date of publication of application: 19.01.2022
(73) Proprietor: Goodgut S.L., 17003 Girona (ES); Fundacio Institut d'Investigació Biomèdica de Girona Dr. Josep Trueta, 17190 Salt (Girona) (ES); Universitat de Girona, 17004 Girona (ES)
(72) Inventor: SERRA PAGÈS, Mariona, 17006 Figueres (Girona) (ES); GARCÍA-GIL, Jesús, 17004 Girona (ES); ALDEGUER MANTÉ, Xavier, 17003 Girona (ES); MALAGÓN RODRIGUEZ, Marta, 17491 Peralada (Girona) (ES); RAMIÓ PUJOL, Sara, 17834 Porqueres (Girona) (ES)
(74) Representative: Hoffmann Eitle
(86) International application number: PCT/EP2020/056575
(87) International publication number: WO 2020/182922

(56) References cited:
- EP-A1- 2 955 232
- WO-A1-2016/161380
- WO-A1-2018/036503
- WO-A2-2012/177639
- J. P. ZACKULAR ET AL: "The Human Gut Microbiome as a Screening Tool for Colorectal Cancer", CANCER PREVENTION RESEARCH, vol. 7, no. 11, 7 August 2014 (2014-08-07), United States, pages 1112 - 1121, XP055333767, ISSN: 1940-6207, DOI: 10.1158/1940-6207.CAPR-14-0129

## Description

### FIELD OF THE INVENTION

The present invention relates to the field of cancer diagnosis. Specifically, it relates to an improved method for the screening, diagnosis and/or monitoring of colorectal advanced neoplasia (AN), advanced adenoma (AA) and/or colorectal cancer (CRC), wherein AN encompasses CRC and AA. In particular, said method provides an increase of specificity due to a reduction of false positive results in the fecal occult blood test (FOBT) using a signature based on bacterial markers. It further relates to the use of said method in the selection of subjects for conducting an exploratory test (e.g., a colonoscopy) or for the treatment with an anti-cancer therapy.

### BACKGROUND OF THE INVENTION

CRC is the third most common cancer in men and the second in women worldwide, and a leading cause of cancer mortality (Estimated Cancer Incidence, Mortality and Prevalence Worldwide in 2012, http://globocan.iarc.fr/Pages/fact_sheets_population.aspx.). It affects 6% of individuals by the age of 75, being the incidence much greater in developed than in developing countries (Wilson, 2005). Although cancers show a strong hereditary component, most of CRC are sporadic and their development is slow (Brenner et al, 2014). In most patients, there is absence of symptomatology until advanced disease is present. Regular bowel cancer screening has been shown to reduce the risk of dying from CRC by 26%, when a fecal test is used, and up to 50% when flexible sigmoidoscopy is applied (Zauber, 2015; Elmunzer et al, 2012). However, CRC screening programs are only implemented in some countries around the world.

Guidelines recommend routine screening for CRC in asymptomatic adults starting at age 50 (Rex et al, 2017). Current screening programs are based on two strategies: invasive procedures based on endoscopy examinations and non-invasive procedures based on fecal tests (Quintero et al, 2012). There are different endoscopy procedures for CRC screening such as flexible sigmoidoscopy and colonoscopy. The main advantage of colonoscopy is that it enables direct visualization of the whole colon making it possible to perform an accurate diagnostic and, therefore, to prevent CRC through the early detection of precancerous lesions (Young & Womeldorph, 2013). Nevertheless, this procedure requires bowel preparation and sedation, there is risk of bowel perforation and other adverse effects, and it is time-consuming and expensive (Rutter et al, 2012; Sieg et al, 2001). Flexible sigmoidoscopy is an alternative approach that enables the direct visualization of the distal colon, that is, rectum, sigmoid and descendent colon. This method avoids sedation and reduces risk of bowel perforation (Gatto et al, 2003). Fecal tests are becoming the preferred CRC screening procedure in most countries due to its non-invasiveness and the lower costs compared to endoscopy strategies. Subjects with a positive result of any of the alternative fecal test generally undergo a colonoscopy in order to make an accurate diagnosis. Therefore, non-invasive tests are oftentimes used as a preliminary step in the screening approach.

One of the fecal tests used in screening programs is the fecal occult blood test that uses guaiac methods (guaiac Fecal Occult Blood Test, gFOBT), which are based on a chemical oxidation reaction between heme and alpha guaionic acid. The main disadvantage of these kind of tests is the requirement of a prescribed diet in order to avoid false-positive results that can occur because of the consumption of specific foods, alcohol or non-steroidal anti-inflammatory drugs (Sanford, 2009). Despite its non-invasiveness, it has shown to have a low sensitivity for CRC (25-38%) and for pre-cancerous lesions (16-31%) (Stracci, 2014). Fecal immunochemical test (FIT) was developed to overcome gFOBT low sensitivity. This test is specific for human blood hemoglobin and does not require dietary restrictions. Several studies have demonstrated a higher sensitivity of FIT screening compared to gFOBT (Brenner & Tao, 2013; Shapiro et al, 2017; Mousavinezhad et al, 2016; Goede et al, 2017). Although overall sensitivity of FIT for CRC is around 61-91% and for advanced adenomas between 27-67% (Stracci et al, 2014), these figures still imply a high false-positive rate.

Recently, it has been reported that bacterial communities in the intestinal mucosa of CRC patients are different from those of healthy individuals (Borges-Canha et al, 2015; Mira-Pascual et al, 2014). Evidences suggest that gut microbiota may play an important role in CRC pathogenesis (Sobhani et al, 2011; Jun et al, 2010). To date, two possible mechanisms through which gut microbiota could induce tumorigenesis have been described. On the one hand, gut microbiota may promote chronic inflammation which in turn can lead to tumor formation (Kostic et al, 2013; Zackular et al, 2014). On the other hand, it has been evidenced that some dietary components metabolized by gut microbiota, such as red meat, result in the production of carcinogenic compounds (Joshi et al, 2015). Moreover, a number of recent studies have been done to elucidate whether or not there is a CRC specific dysbiosis, or any particular species that can be associated to CRC development (Dulal & Keku, 2014; Zeller et al, 2014; Marchesi et al, 2011).

In 2012, a preliminary, prospective study performed by our group with 60 individuals (41 CRC patients and 19 patients with normal colonoscopy) defined a bacterial cluster in mucosal biopsies which prevalence was correlated with CRC risk (Mas de Xaxars Rivero, 2012). From the 55 phylotypes analyzed, 6 showed significantly higher frequencies in CRC patients than in control subjects. Five of them shared similarity with uncultured bacterial sequences retrieved from the human gastrointestinal tract or human feces, and one shared 97% similarity with *Parabacteroides merdae.* In contrast, there were two phylotypes, B34 (99% similarity to *Clostridium nexile*) and B35 (97% similarity to *Roseburia faecalis*), which were more prevalent in healthy subjects than in CRC patients.

Subsequently, the inventors' group designed quantitative polymerase chain reaction systems (qPCR) specifically targeted to those bacterial markers (Mas de Xaxars Rivero, 2012). Bacterial signatures were later tested on stool samples (7 from healthy controls and 9 CRC patients) looking for different abundances to check which one was suitable to be used as a non-invasive tool for CRC screening (WO 2015/132273). A retrospective clinical study including 46 patients of the Hospital Universitari de Girona Dr. Josep Trueta (Girona, Spain) confirmed the suitability of some bacterial signatures as CRC markers (WO 2015/132273).

EP2955232 discloses a method of screening, diagnosis and/or monitoring of adenomas and/or colorectal cancer (CRC) in a subject comprising quantifying in a feces sample isolated from said subject the fecal metagenome comprising the species:
i) Fusobacterium nucleatum subsp. vincentii, Fusobacterium nucleatum subsp. animalis, Porphyromonas asaccharolytica, and Peptostreptococcus stomatis, and/or
ii) Enterobacteriaceae bacterium 9_2_54FAA, Acidaminococcus intestini, Bacteroides stercoris,and Ruminococcaceae bacterium D16,and optionally Bacteroides fragilis.

It further describes a combination test comprising an FOBT test (i.e., the Hemoccult FOBT test) and the metagenome test.

Despite recent advances, there is still the need to find new tools for the early detection of advanced colorectal neoplasia through population-based screening and surveillance strategies in order to reduce CRC mortality. The ideal technique should be non-invasive, cost-effective, reproducible and capable to detect premalignant lesions with high risk of tumor development and high sensitivity and specificity values.

### SUMMARY OF THE INVENTION

The present invention provides a new non-invasive AA, CRC and/or AN screening, diagnosis and/or monitoring test based on a new bacterial signature that complements fecal occult blood tests (e.g., FIT) and is able to reduce FOBT-associated false positive results and thus increase its specificity and positive predictive value (PPV) while maintaining similar levels of sensitivity.

The first aspect of the invention relates to a method for increasing the specificity or reducing the false positive rate of a fecal occult blood test (FOBT) for the screening, diagnosis and/or monitoring of colorectal advanced neoplasia (AN), advanced adenoma (AA) and/or colorectal cancer (CRC) in a subject, wherein said method comprises:
a) optionally, conducting a FOBT in a feces sample isolated from said subject, wherein said FOBT comprises the determination of presence of occult blood in said feces sample; and
b) quantifying in an intestinal sample (preferably a feces sample) isolated from said subject at least one of the following bacterial markers, or any combination thereof:
   - *Gemella morbillorum* (GMLL);
   - *Peptostreptococcus stomatis* (PTST), and
   - *Bacteroides fragilis* (BCTF).

In a related aspect, the invention pertains to a method for the screening, diagnosis and/or monitoring of AN, AA and/or CRC in a subject, said method comprising:
a) optionally, conducting a FOBT in a feces sample isolated from said subject, wherein said FOBT comprises the determination of presence of occult blood in said feces sample;
b) quantifying in an intestinal sample (preferably a feces sample) isolated from said subject at least one of the following bacterial markers, or any combination thereof:
   - *Gemella morbillorum* (GMLL);
   - *Peptostreptococcus stomatis* (PTST), and
   - *Bacteroides fragilis* (BCTF).

In a further aspect, the present invention provides a method of treating a subject having AN, AA and/or CRC, wherein said subject has been selected by a method for the screening, diagnosis and/or monitoring of other aspects of the invention, and wherein said method further comprises administering to the subject an anti-cancer therapy.

In a related aspect, the present invention also provides any of the methods as described in other aspects, which further comprises a step of administering to said patient a therapeutically effective amount of an anti-cancer therapy.

In another aspect, the invention relates to an anti-cancer therapy for use in a method for treatment of a cancer patient, wherein said cancer patient has been selected by a screening, diagnostic and/or monitoring method of other aspects of the invention.

In an additional aspect, the invention also refers to a method for selecting a subject for conducting an exploratory test selected from the group consisting of colonoscopy, flexible sigmoidoscopy, double-contrast barium enema and computed tomography (CT) colonography, preferably for conducting a colonoscopy, wherein said subject has been selected by a screening, diagnosis or monitoring method of other aspects of the invention.

In still a further aspect, the invention pertains to a method for conducting an exploratory test in a subject, wherein said subject has been selected by a screening, diagnosis or monitoring method of other aspects of the invention, wherein said method further comprises conducting an exploratory test to the subject, wherein the test is selected from the group consisting of colonoscopy, flexible sigmoidoscopy, double-contrast barium enema and computed tomography (CT) colonography, preferably a colonoscopy.

In another aspect, the invention provides a kit suitable for quantifying any of GMLL, PTST, BCTF or BCTT, wherein said kit comprises one or more of the following reagents:
i. an oligonucleotide specific to GMLL genome, preferably an oligonucleotide specific to SEQ ID NO:1 ;
ii. an oligonucleotide specific to PTST genome, preferably an oligonucleotide specific to SEQ ID NO:2;
iii. an oligonucleotide specific to BCTF genome, preferably an oligonucleotide specific to SEQ ID NO:3 ;
iv. an oligonucleotide specific to BCTT genome, preferably an oligonucleotide specific to SEQ ID NO:4; and
v. optionally, an oligonucleotide suitable for the quantification of Eubacteria as defined herein above.

In a further aspect, the present invention also provides a kit as described herein for use in a method for the screening, diagnosis and/or monitoring of AN, AA and/or CRC as described herein.

### BRIEF DESCRIPTION OF THE DRAWINGS

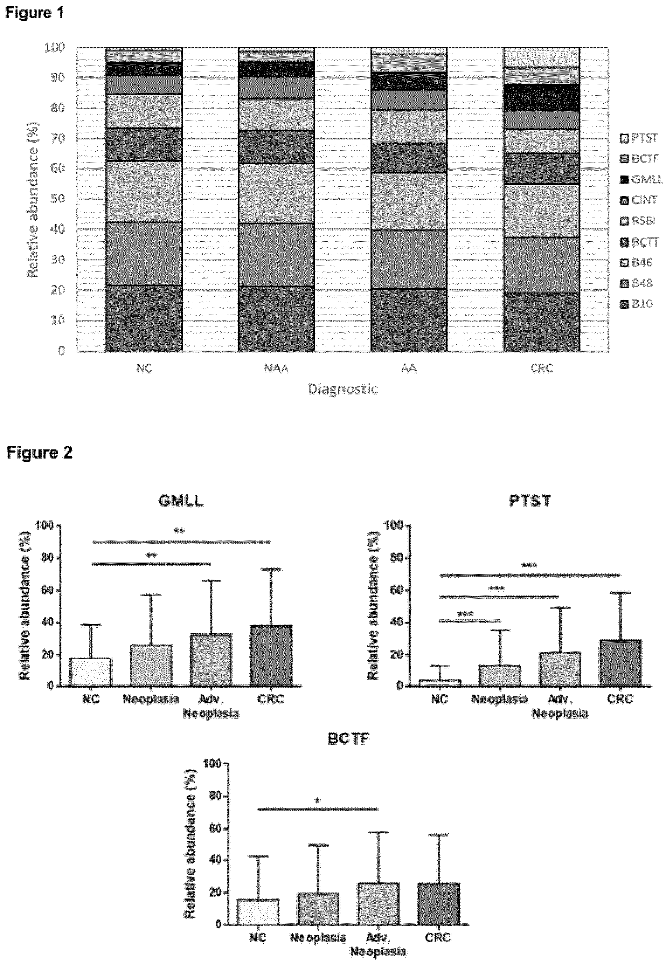

Figure 1: Relative abundance in percentage of the analyzed biological markers (B10 (best match BLAST for *Faecalibacterium prausnitzii,* B46 (best match BLAST for *Faecalibacterium prausnitzii, Subdoligranulum variabil*), B48 (best match BLAST for *Ruminococcus, Roseburia, Coprococcus*), *Gemella morbillorum* (GMLL), *Peptostreptococcus stomatis* (PTST), *Bacteroides fragilis* (BCTF), *Collinsella intestinalis* (CINT), *Bacteroides thetaiotaomicron* (BCTT) and *Roseburia intestinalis* (RSBI)); for subjects with normal colonoscopy (NC), non-advanced adenoma (NAA), advanced adenoma (AA), and colorectal cancer (CRC).
Figure 2: Biomarkers abundances comparison among different diagnostics. NC, normal colonoscopy; neoplasia, non-advanced adenoma + advanced adenoma + colorectal cancer; advanced neoplasia, advanced adenoma + colorectal cancer; CRC, colorectal cancer. Level of significance: * when p-value<0.05, ** when p-value<0.01 and *** when p-value<0.001.

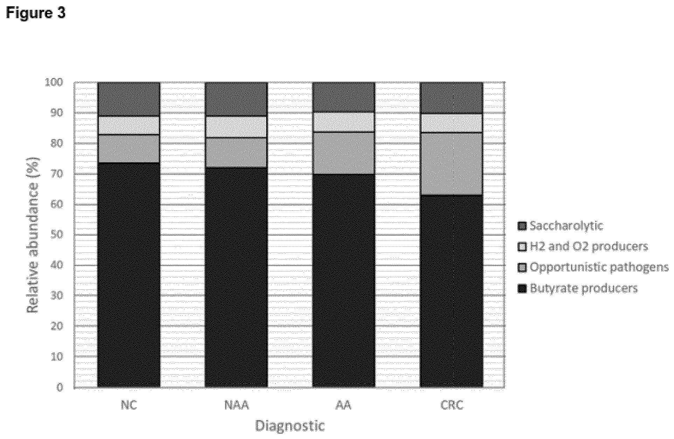

Figure 3: Relative abundance in percentage of the analyzed biological markers (butyrate producing species: B10, B46, B48, and *Roseburia intestinalis* (RSBI); opportunistic pathogens: *Gemella morbillorum* (GMLL), *Peptostreptococcus stomatis* (PTST), and *Bacteroides fragilis* (BCTF); H₂ and O₂ producers: *Collinsella intestinalis* (CINT); and saccharolytic bacteria: *Bacteroides thetaiotaomicron* (BCTT)) for subjects with normal colonoscopy (NC), non-advanced adenoma (NAA), advanced adenoma (AA), and colorectal cancer (CRC).

### DETAILED DESCRIPTION OF THE INVENTION

### Definitions

The terms "subject", or "individual‴ are used herein interchangeably to refer to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race.

The term "sensitivity" as used herein refers to the proportion of subjects who have the target condition (reference standard positive) and give positive test results (TP/ (TP + FN)). It shows how good the test is at detecting a disease. Sensitivity ("sens") may be within the range of 0 (0%) < sens < 1 (100%) and ideally, the number of false negatives equaling zero or close to equaling zero and sensitivity equaling one (100%) or close to equaling one (100%).

The term "specificity" as used herein in connection with a test refers to the proportion of subjects without the target condition (reference standard negative) and give negative test results (TN/ (TN + FP)). It shows how good the test is at identifying normal (negative) condition. Specificity ("spec") may be within the range of 0 (0%) < spec < 1 (100%) and ideally, the number of false positives equaling zero or close to equaling zero and specificity equaling one (100%) or close to equaling one (100%).

The term "accuracy" as used herein refers to the proportion of true results, either true positive or true negative, in a population. It measures the degree of veracity of a screening test on a condition, i.e., how correct is the determination and exclusion of a given condition (TN + TP)/(TN+TP+FN+FP). Accuracy ("acc") may be within the range of 0 (0%) < acc < 1 (100%) and ideally, the number of false positives and false negative equaling zero or close to equaling zero and accuracy equaling one (100%) or close to equaling one (100%).

The term "Receiver Operating Characteristic (ROC) curves" as used herein refers to a graphical plot that illustrates the performance of a binary classifier system as its discrimination threshold is varied. The curve is created by plotting the true positive rate against the false positive rate at various threshold settings. The true positive rate is also known as sensitivity. The false positive rate is calculated as 1 - specificity. The ROC curve is thus a way of graphically displaying the true positive rate versus the false positive rate (sensitivity vs (1-specificity)) across a range of cut-offs and of selecting the optimal cut-off for clinical use. Accuracy expressed as the area under the ROC curve (AUC) provides a useful parameter for comparing test performance. An AUC approaching 1 indicates that the test is highly sensitive as well as highly specific whereas an AUC approaching 0.5 indicates that the test is neither sensitive nor specific. In general, a test is considered to be a suitable discriminator if the AUC is from 0.6 to 0.75, to have good discrimination capacity if the AUC is from 0.75 to 0.9 and to be an excellent discriminator if the AUC is from 0.9 to 1. For further details see for instance, Zweig MH and Campbell G, Clinical Chemistry 1993; 39:561-577 or Greiner et al. Preventive Veterinary Medicine 2000; 45:23-41.

The term "probe" as used herein refers to synthetic or biologically produced nucleic acids, between 10 and 285 base pairs in length which contains specific nucleotide sequences that allow specific and preferential hybridization under predetermined conditions to target nucleic acid sequences, and optionally contains a moiety for detection or for enhancing assay performance. A minimum of ten nucleotides is generally necessary in order to statistically obtain specificity and to form stable hybridization products, and a maximum of 285 nucleotides generally represents an upper limit for length in which reaction parameters can be easily adjusted to determine mismatched sequences and preferential hybridization. Probes may optionally contain certain constituents that contribute to their proper or optimal functioning under certain assay conditions. For example, probes may be modified to improve their resistance to nuclease degradation (e.g., by end capping), to carry detection ligands (e.g., fluorescein), to carry ligands for purification or enrichment purposes (e.g. biotin) or to facilitate their capture onto a solid support (e.g., poly-deoxyadenosine "tails").

The term "primers" as used herein refers to oligonucleotides that can be used in an amplification method, such as a polymerase chain reaction ("PCR"), to amplify a nucleotide sequence. Primers are designed based on the polynucleotide sequence of a particular target sequence, e.g., one specific 16S rDNA sequence.

The term "specific" as used herein in connection with a nucleotide sequence means that a nucleotide sequence will hybridize to/amplify a predetermined target sequence and will not substantially hybridize to/amplify a non-target sequence under the assay conditions, generally stringent conditions are used.

The term "hybridization" as used herein refers to a process by which, under predetermined reaction conditions, two partially or completely complementary strands of nucleic acid are allowed to come together in an antiparallel fashion to form a double-stranded nucleic acid with specific and stable hydrogen bonds, following explicit rules pertaining to which nucleic acid bases may pair with one another.

The term "substantial hybridization" means that the amount of hybridization observed will be such that one observing the results would consider the result positive with respect to hybridization data in positive and negative controls. Data which is considered "background noise" is not substantial hybridization.

The term "stringent hybridization conditions" means approximately 35 °C to 65 °C in a salt solution of approximately 0.9 molar NaCl. Stringency may also be governed by such reaction parameters as the concentration and type of ionic species present in the hybridization solution, the types and concentrations of denaturing agents present, and the temperature of hybridization. Generally, as hybridization conditions become more stringent, longer probes are preferred if stable hybrids are to be formed. As a rule, the stringency of the conditions under which hybridization is to take place will dictate certain characteristics of the preferred probes to be employed.

The term "therapeutically effective amount" as used herein refers to an amount that is effective, upon single or multiple dose administration to a subject (such as a human patient) in the prophylactic or therapeutic treatment of a disease, disorder or pathological condition.

The term "anti-cancer therapy" as used herein refers to a therapy useful in treating cancer. Examples of anti-cancer therapies include, but are not limited to, e.g., surgery, chemotherapeutic agents, growth inhibitory agents, cytotoxic agents, anti-hormonal agents, agents used in radiation therapy, anti-angiogenesis agents, apoptotic agents, anti-tubulin agents, and other agents to treat cancer, such as anti-HER-2 antibodies (e.g., Herceptin^{®}), anti-CD20 antibodies, an epidermal growth factor receptor (EGFR) antagonist (e.g., a tyrosine kinase inhibitor), HER1/EGFR inhibitor (e.g., erlotinib (Tarceva^{®})), platelet derived growth factor inhibitors (e.g., Gleevec^{™} (Imatinib Mesylate)), a COX-2 inhibitor (e.g., celecoxib), interferons, cytokines, antagonists (e.g., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also included in the invention.

The term "cytotoxic agent" as used herein refers to a substance that inhibits or prevents the function of cells and/or causes destruction of cells. The term is intended to include radioactive isotopes (e.g. At<211>, I<131>, I<125>, Y<90>, Re<186>, Re<188>, Sm<153>, Bi<212>, P<32>and radioactive isotopes of Lu), chemotherapeutic agents, and toxins such as small molecule toxins or enzymatically active toxins of bacterial, fungal, plant or animal origin, including fragments and/or variants thereof.

The term "chemotherapeutic agent" as used herein refers to a chemical compound useful in the treatment of cancer. Examples of chemotherapeutic agents include alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1 and calicheamicin omegal1; dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2'2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel, ABRAXANE^{®} Cremophor-free, albumin-engineered nanoparticle formulation of paclitaxel, and TAXOTERE^{®} doxetaxel; chloranbucil; GEMZAR^{®} gemcitabine; 6-thioguanine; mercaptopurine; methotrexate; platinum analogs such as cisplatin, oxaliplatin and carboplatin; vinblastine; platinum; etoposide (VP-16); ifosfamide; mitoxantrone; vincristine; NAVELBINE^{®} vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluorometlhylornithine (DMFO); retinoids such as retinoic acid; capecitabine; combretastatin; leucovorin (LV); oxaliplatin, including the oxaliplatin treatment regimen (FOLFOX); lapatinib (Tykerb^{®}); inhibitors of PKC-alpha, Raf, H-Ras, EGFR (e.g., erlotinib (Tarceva^{®})) and VEGF-A that reduce cell proliferation and pharmaceutically acceptable salts, acids or derivatives of any of the above.

The term "anti-hormonal agents" as used herein refers to agents that act to regulate or inhibit hormone action on tumors such as anti-estrogens and selective estrogen receptor modulators (SERMs), including, for example, tamoxifen (including NOLVADEX^{®} tamoxifen), raloxifene, droloxifene, 4-hydroxytamoxifen, trioxifene, keoxifene, LY 117018, onapristone, and FARESTON· toremifene; aromatase inhibitors that inhibit the enzyme aromatase, which regulates estrogen production in the adrenal glands, such as, for example, 4(5)-imidazoles, aminoglutethimide, MEGASE^{®} megestrol acetate, AROMASIN^{®} exemestane, formestanie, fadrozole, RIVISOR^{®} vorozole, FEMARA^{®} letrozole, and ARIMIDEX^{®} anastrozole; and antiandrogens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in aberrant cell proliferation, such as, for example, PKC-alpha, Ralf and H-Ras; ribozymes such as a VEGF expression inhibitor (e.g., ANGIOZYME^{®} ribozyme) and a HER2 expression inhibitor; vaccines such as gene therapy vaccines, for example, ALLOVECTIN^{®} vaccine, LEUVECTIN^{®} vaccine, and VAXID^{®} vaccine; PROLEUKIN^{®} rIL-2; LURTOTECAN^{®} topoisomerase 1 inhibitor; ABARELIX^{®} rmRH; and pharmaceutically acceptable salts, acids or derivatives of any of the above.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); epidermal growth factor; hepatic growth factor; fibroblast growth factor; prolactin; placental lactogen; tumor necrosis factor-alpha and -beta; mullerianinhibiting substance; mouse gonadotropin-associated peptide; inhibin; activin; vascular endothelial growth factor; integrin; thrombopoietin (TPO); nerve growth factors such as NGFalpha; platelet-growth factor; transforming growth factors (TGFs) such as TGF-alpha and TGFbeta; insulin-like growth factor-I and -II; erythropoietin (EPO); osteoinductive factors; interferons such as interferon-alpha, -beta and -gamma colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; a tumor necrosis factor such as TNF-alpha or TNF-beta; and other polypeptide factors including LIF and kit ligand (KL). As used herein, the term cytokine includes proteins from natural sources or from recombinant cell culture and biologically active equivalents of the native sequence cytokines.

The term "growth inhibitory agent" as used herein refers to a compound or composition which inhibits growth of a cell in vitro and/or in vivo. Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), TAXOL^{®}, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13 .

The term "radiation therapy" or "radiotherapy" as used herein refers to the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one-time administration and typical dosages range from 10 to 200 units (Grays) per day.

The term "intestinal disease" as used herein refers to those diseases or disorders affecting the small intestine, the colon and/or rectum.

### The methods of the invention

In a first aspect, the present invention relates to a method for increasing the specificity or reducing the false positive rate of a fecal occult blood test (FOBT) for the screening, diagnosis and/or monitoring of colorectal advanced neoplasia (AN), advanced adenoma (AA) and/or colorectal cancer (CRC) in a subject, wherein said method comprises:
a) optionally, conducting a FOBT in a feces sample isolated from said subject, wherein said FOBT comprises the determination of presence of occult blood in said feces sample; and
b) quantifying in an intestinal sample (preferably a feces sample) isolated from said subject at least one of the following bacterial markers, or any combination thereof:
   - *Gemella morbillorum* (GMLL);
   - *Peptostreptococcus stomatis* (PTST), and
   - *Bacteroides fragilis* (BCTF).

In a related aspect, the invention pertains to a method for the screening, diagnosis and/or monitoring of AN, AA and/or CRC in a subject, said method comprising:
a) optionally, conducting a FOBT in a feces sample isolated from said subject, wherein said FOBT comprises the determination of presence of occult blood in said feces sample;
b) quantifying in an intestinal sample (preferably a feces sample) isolated from said subject at least one of the following bacterial markers, or any combination thereof:
   - *Gemella morbillorum* (GMLL);
   - *Peptostreptococcus stomatis* (PTST), and
   - *Bacteroides fragilis* (BCTF).

In preferred embodiments, the FOBT in a) and the quantification of bacterial markers in b) are conducted in a feces sample, preferably in the same feces sample.

The reference strain of GMLL is *Gemella morbillorum* NCTC11323 strain (Bacteria; Firmicutes; Bacilli; Bacillales; Bacillales Family XI. Incertae Sedis; Gemella) and its genomic sequence has GenBank access number: LS483440.1. In particular embodiments, oligonucleotides specific to SEQ ID NO: 1 may be used for the quantification of GMLL. SEQ ID NO: 1 corresponds to GenBank access number: LS483440.1 REGION: 827110..827892.

1 atggcgagtt tattacaaaa aacaagaaaa ataagtacaa ttttacaaga aggacgtcat
61 gataatgtcg attttgaagc aatggcaatg cgcttaagtc ctattttaga ttctgttgtc
121 tatattttag atgtagaagg taatattctt gggtatgatt ctattgtaga ttattctaac
181 gaacgtatgg aagaaattat tcgtgcgagg aaagtgccta aagcttattt agatgcaaca
241 ttaaaagtat atgctacgaa ggttaatatt ccatttcaag atccgctttc tattttccca
301 gatgaagaga aagaaagatt tgatgggaat acctatactg taattttacc aataagaggc
361 gggggagaac gccttggaac tcttgttata ggaagaatgg ataatgactt tcaagatgat
421 gatttggtgt tagcagaata tgcgtcaaca gtagtaggaa ttgaaattct tcacgaaaaa
481 caagataaag aaaaaaatct agctagagac aaagatatgg tgaatatggc tcttaattcg
541 ttatcatatt cagaaaaaga agcaattgaa catattttca gagaattaga tgggacagaa
601 ggactactta tcgcaagtaa aattgcagat agagtaggaa ttactcgctc agttattgtt
661 aatgcactta gaaaattaga aagtgcaggt attatagagt caaaatcatt aggtatgaaa
721 ggtacttata taaaagtact taaagaatat tttttagaat taatgttttc taatgaattt
781 taa

The reference strain of PTST is *Peptostreptococcus stomatis* DSM 17678 strain (Bacteria; Firmicutes; Clostridia; Clostridiales; Peptostreptococcaceae; Peptostreptococcus) and its genomic sequence has GenBank access number: ADGQ01000060.1. In particular embodiments, oligonucleotides specific to SEQ ID NO: 2 may be used for the quantification of PTST. SEQ ID NO: 2 corresponds to GenBank access number: ADGQ01000060.1 REGION: complement (142796..143362).

1 atggatatta ttcaacttag caacagactt atagaataca gaaaaagtaa caacttaact
 61 attaaagatt tctctgacat gtccggaatt agtactgctc tgcttagtca attagaaaga
121 ggtgttggaa accctagcct gagtgtattg aactctatag cagataccat gaatactagc
181 ctctcctctt tattagaaga acctgttgtt ttagaaaacc tggttagaag gtctgacacc
241 ctatccacta ttgtctaccc atgtaggaac aaccttgaat ttcagttgct cacaactagg
301 tctactactc agagcatcaa ccttgtaaga ataatatttc atgctcattc agaaactaat
361 taccagccac ttggaaaaac cgtttctgat gatataatcc acattgaaaa gggcagtatt
421 attgttcaaa ctgatgatgg taagtctatt caactaaaaa aaggtgatac tatgaggata
481 ccacctgacc ttaggtataa attcaagaat atttctgtac acaaggcaca tatgatttgt
541 gccactaata aacttgaagt aagataa

The reference strain of BCTF is *Bacteroides fragilis* NCTC 9343 strain (Bacteria; Bacteroidetes; Bacteroidia; Bacteroidales; Bacteroidaceae; Bacteroides) and its genomic sequence has GenBank access number: CR626927.1. In particular embodiments, oligonucleotides specific to SEQ ID NO: 3 may be used for the quantification of BCTF. SEQ ID NO: 3 corresponds to GenBank access number: CR626927.1 REGION: 417101..417817.

1 atggcaaata ctaatatgga acacggagaa ataatactat accaaccaga caatactata
61 aaactggaag tgcgaataga gaatgaaacc gtttggttga cacaagcaca aattgttaac
121 ttattccagt caagtaaagc caatatcagt gagcatataa gaaatatata tgactcagat
181 gaattatctg ctgaatcaac tgttcggaaa ttccgaacag ttcgaatgga aggtaataga
241 aaggttaccc gcattcttga atattataat ctggatatga ttatttccgt aggttatcgt
301 gtgaattcca agcgaggagt ccagttccgc caatggtcaa caggagtact taaagaatat
361 ctattaaaag gctacgcaat caatcagcgc gtggagcagt tggaaaacaa agcaaacacc
421 catgaccggc aactggaaga gttaacaaac aaagtcgact ttttcgtccg gacttcatta
481 cctcctattg aaggtgtctt tttcaacgga cagattttcg atgcctatgt cttttccgct
541 cagttgataa agtctgcaaa gtcatcttta gtattgattg acaactttgt cgatgaaagc
601 gtgctcttac tattgagtaa acgtttgccc ggagtgactt ctatcatata taccaagcaa
661 gtaactccac agttagaatt agatttgaca aagcacaaca gtcaataccc ccaatag

In preferred embodiments, these specific oligonucleotides are any of those recited in Table 3 for GMLL, PTST and BCTF, respectively, and sequences with at least 80% identity to any thereof.

As shown in Example 3, PTST was found to be highly correlated with neoplasia lesions (non-advanced adenoma + advanced adenoma + CRC; p<0.001). Moreover, each of GMLL, PTST, and BCTF were identified as potential biomarkers for the detection of advanced neoplasia (AN) lesions (p=0.006, p<0.001, and p=0.030, respectively). Also, it was found that GMLL and PTST were significantly more abundant in CRC than in healthy subjects (p=0.004, and p<0.001, respectively).

As used herein, the term "colorectal cancer", **"CRC"** refers to cancer that starts in the colon or the rectum. These cancers can also be referred to separately as colon cancer or rectal cancer, depending on where they start. Colon cancer and rectal cancer have many features in common. According to the ACS, several types of cancer can start in the colon or rectum. More than 95% of colorectal cancers are a type of cancer known as adenocarcinomas. These cancers start in cells that form glands that make mucus to lubricate the inside of the colon and rectum. Other, less common types of tumors may also start in the colon and rectum. These include: carcinoid tumors, gastrointestinal stromal tumors (GISTs), lymphomas and sarcomas. In a preferred embodiment, said colorectal cancer is adenocarcinoma.

Adenoma is a non-cancerous growth of abnormal glandular cells on the inner lining of an organ such as the colon. Adenomas that meet one or more of the following characteristics: measuring 10 mm or more in diameter, with villous architecture, high-grade dysplasia, or intramucosal carcinoma, are typically classified as **advanced adenomas** (Quintero E., Castells A., et al., N Engl J Med. 2012, 366(8):697-706; Cubiella J. et al., Cancer Epidemiol Biomarkers Prev. 2014, 23(9):1884-92; Muto T, Bussey HJR MB., Cancer 1975;36:2251-70).

The term colorectal **advanced neoplasia** (AN) as used herein encompasses CRC and advanced adenoma (AA). According to the ACS, tests that have the best chance of finding both AA and CRC are preferred. Cohort studies involving patients with adenomas suggested that polypectomy can prevent approximately 80% of colorectal cancers (Citarda F, et al., Gut 2001, 48:812-5; Winawer SJ, et al., N Engl J Med 1993, 329:1977-81).

The term **"diagnosis",** as used herein, refers both to the process of attempting to determine and/or identify a possible disease in a subject, i.e. the diagnostic procedure, and to the opinion reached by this process, i.e. the diagnostic opinion. As such, it can also be regarded as an attempt at classifying an individual's condition into separate and distinct categories that allow medical decisions about treatment and prognosis to be made. It is to be understood that the method, in a preferred embodiment, is a method carried out *in vitro,* i.e. not practiced on the human or animal body. In particular, the diagnosis to determine CRC, AA and/or AN patients, may relate to the capacity to identify and classify CRC, AA and/ or AN patients.

In a particular embodiment, the method of diagnosis of the invention is carried out in a subject who is suspected of having CRC. The term "subject suspected of having CRC" as used herein refers to a subject that presents one or more signs or symptoms which may be indicative of CRC. A subject suspected of having CRC, further encompasses an individual who has received a preliminary diagnosis but for whom a confirmatory test (e.g., a colonoscopy) has not yet been done.

Signs or symptoms which may be indicative of CRC include for instance one or more of the following: unexplained weight loss, abdominal pain, unexplained rectal bleeding, iron-deficiency anemia, changes in bowel habit, and presence of occult blood in feces (see for example, Jellema et al, BMJ 2010, 340: c1269, or Adelstein et al. BMC Gastroenterology 2011, 11:65). For illustrative purposes, are provided herein below those instances where CRC is suspected in the current NICE guideline (NG12); https://www.nice.org.uk/guidance/ng12/chapter/1-Recommendations-organised-by-site-of-cancer#lower-gastrointestinal-tract-cancers:
- Refer adults using a suspected cancer pathway referral (e.g., for an appointment within 2 weeks) for colorectal cancer if:
   - they are aged 40 and over with unexplained weight loss and abdominal pain or
   - they are aged 50 and over with unexplained rectal bleeding or
   - they are aged 60 and over with:
      ∘ iron-deficiency anemia or
      ∘ changes in their bowel habit, or
   - tests show occult blood in their feces.
- Consider a suspected cancer pathway referral (e.g., for an appointment within 2 weeks) for colorectal cancer in adults with a rectal or abdominal mass.
- Consider a suspected cancer pathway referral (e.g., for an appointment within 2 weeks) for colorectal cancer in adults aged under 50 with rectal bleeding and any of the following unexplained symptoms or findings:
   - abdominal pain
   - change in bowel habit
   - weight loss
   - iron-deficiency anemia.
- Offer testing for occult blood in feces to assess for colorectal cancer in adults without rectal bleeding who:
   - are aged 50 and over with unexplained:
      ∘ abdominal pain or
      ∘ weight loss, or
   - are aged under 60 with:
      ∘ changes in their bowel habit or
      ∘ iron-deficiency anemia, or
   - are aged 60 and over and have anemia even in the absence of iron deficiency.

The method of the invention may also be used for the differential diagnosis between CRC, advanced adenoma and/or advanced neoplasia with respect to non-advanced adenoma.

The most effective and economic measure to reduce CRC incidence and mortality are CRC risk screening and monitoring tests. Screening tests are grouped into those that primarily detect cancer early; and those that can detect cancer early and also can detect advanced adenoma, thus providing a greater potential for prevention through polypectomy (i.e., polyps removal), see American Cancer Society (ACS) 2008 screening and surveillance guidelines, Levin et al. (CA Cancer J Clinicians, 2008;58(3)130-160). Different screening guidelines being of application at people of average risk and at people with increased or high risk (http://www.cancer.org/cancer/colonandrectumcancer/moreinformation/colonandrectumcancere arlydetection/colorectal-cancer-early-detection-acs-recommendations).

The term **"screening"** is understood herein as the examination or testing of a group of asymptomatic individuals pertaining to the general population, or of a group of individuals having one or more risk factors (e.g., a subject with intermediate or high risk of developing a disease), with the objective of discriminating healthy individuals from those who have or are suspected of having a disease. A method of screening is generally used for the "early detection" of a disease. The expression "early detection" refers to detection before the presence of clinical signs.

The goal of cancer screening is to reduce mortality through early detection and treatment thus enabling a reduction in incidence of advanced disease which generally has a worse prognosis. To this end, modern CRC screening can achieve this goal through the detection of adenocarcinomas (preferably early-stage adenocarcinomas) and the detection and removal of advanced adenoma. The method of screening of the invention is thus found to be a potent tool to reduce mortality associated to CRC through detection of CRC and/or advanced neoplasia. The method of the screening of the invention may be conducted in individuals that do not present signs and/or symptoms of CRC (referred herein as "asymptomatic individuals"). It may also be carried out in subjects with or without personal or family history of colonic neoplasm, or other risk factors of CRC as described below.

In a particular embodiment, the screening method of the invention is carried out in subjects with intermediate CRC risk. These are typically subjects aged 50 years and older without personal or familial CRC background. Average risk women and men aged 50 years and older are encouraged to follow colorectal cancer screening tests (see Table 2 of Levin et al., CA Cancer J Clinicians, 2008:58(3)130-160.

In another embodiment, the screening method of the invention is carried out in increased and/or high-risk subjects. According to the ACS (see Table 3 of Levin et al., CA Cancer J Clinicians, 2008:58(3)130-160, increased and high-risk subjects may include the following:
Increased risk
   - subjects with a history of polyps on prior colonoscopy;
   - subjects with colorectal cancer; and
   - subjects with a family history of colorectal cancer or adenomatous polyps, including:
      i. either CRC or adenomatous polyps in a first degree relative before age 60 years or in 2 or more first-degree relatives at any age; and/or
      ii. either CRC or adenomatous polyps in a first degree relative ≥ age 60 years or in 2 second-degree relatives with CRC
High risk
   - subjects with familial adenomatous polyposis (FAP) diagnosed by genetic testing, or suspected FAP without genetic testing;
   - subjects with hereditary non-polyposis colon cancer (HNPCC or Lynch syndrome), or at increased risk of HNPCC based on family history without genetic testing; and
   - subjects with Inflammatory bowel disease, such as chronic ulcerative colitis or Crohn's disease.

Another goal of the present invention may be to provide a pre-diagnosis tool. Specifically, the methods of the invention may be used for screening and/or identifying subjects with predisposition or an increased risk of having CRC, for instance by detecting the presence of advanced adenoma.

The term "monitoring" as used herein refers to determining the evolution of the disease and/or the efficacy of a surgical and/or therapeutic treatment, for example determining whether there is a remission of the disease; or on the contrary whether there is disease progression or a relapse. One of the goals of the method of monitoring of the invention is to early detect relapses. In a particular embodiment, the method of the invention is for the monitoring of subjects suffering from cancer which have been submitted to surgical resection, optionally in combination with adjuvant and/or neo-adjuvant therapy.

In preferred embodiments, the methods of the invention are for the screening, diagnosis and/or monitoring of AN, AA and/or CRC.

The term "sample" or "biological sample", as used herein, refers to biological material isolated from a subject. The biological sample may contain any biological material suitable for detecting the desired biomarker and may comprise cellular and/or non-cellular material from the subject.

In a preferred embodiment, the samples are intestinal samples. These may be for instance a biopsy from the mucosal tissue of the colon and/or rectum. Preferably, this intestinal sample is a feces sample. Feces samples are routinely used in the clinical practice and a person skilled in the art will know how to identify the most appropriate means for their obtaining and preservation. Once a sample has been obtained, it may be used fresh, it may be frozen or preserved using appropriate means.

The methods of the invention optionally comprise in **step a)** conducting a FOBT in a feces sample isolated from a subject, wherein said FOBT comprises the determination of presence of occult blood in a feces sample isolated from said subject. Blood in the stool is a non-specific finding which may be originated from CRC or larger (> 1 to 2 cm) polyps. Because small adenomatous polyps do not tend to bleed and bleeding from cancers or large polyps may be intermittent or simply not always detectable in a single sample of stool, the proper use of stool blood tests typically requires annual testing that consists of collecting specimens (2 or 3, depending on the product) from consecutive bowel movements. Stool blood tests are conventionally known as fecal occult blood tests **(FOBT)** because they are designed to detect the presence of occult blood in stool. FOBT fall into two primary categories based on the detected analyte: gFOBT and FIT. Guaiac-based tests detect blood in the stool through the pseudoperoxidase activity of heme or hemoglobin, while immunochemical-based tests react to human globin (Levin et al. (CA Cancer J Clinicians, 2008;58(3)130-160).

The usual gFOBT protocol consists of collecting 2 samples from each of 3 consecutive bowel movements. Since the gFOBT is a qualitative test, it is generally visually read by trained laboratory technicians using the naked eye to interpret a visual result. Prior to testing with a sensitive guaiac-based test, individuals usually will be instructed to avoid aspirin and other nonsteroidal anti-inflammatory drugs, vitamin C, red meat, poultry, fish, and some raw vegetables because of diet-test interactions that can increase the risk of both false-positive and false-negative (specifically, vitamin C) results. An example of commercially available gFOBT is Hemoccult SENSA (Beckman Coulter, US).

FIT detects human globin, a protein that along with heme constitutes human hemoglobin. Thus, FIT is more specific for human blood than guaiac-based tests, which rely on detection of peroxidase in human blood and also react to the peroxidase that is present in dietary constituents such as rare red meat, cruciferous vegetables, and some fruits. Further, unlike gFOBT, FIT is not subject to false-negative results in the presence of high-dose vitamin C supplements, which block the peroxidase reaction. In addition, because globin is degraded by digestive enzymes in the upper gastrointestinal tract, FIT is also more specific for lower gastrointestinal bleeding, thus improving their specificity for CRC. Finally, the sample collection for some variants of FIT are less demanding of patients than gFOBT, requiring fewer samples or less direct handling of stool. A variety of FIT exist based on different detection technologies (e.g., reversed passive hemagglutination (RPHA), latex agglutination, ELISA and immunonephelometry) and are encompassed herein (Levin B et al., Gastroenterology 2008;134:1570-95). Preferably, said immunologic test is based on a latex agglutination reaction, such as the OC-SENSOR assay (Eiken Chemical Co., Japan) used in the Examples.

Preferably, said FOBT is a fecal immunochemical test (FIT) quantifying human hemoglobin (hHb). A subject will be determined to be FIT positive when the levels of hHb in the feces sample are above a pre-defined threshold. For instance, a subject may be determined to be FIT positive when hHb levels are equal or above 10 µg hHb/g of feces (or equal or higher than 50 ng of hemoglobin per mL; FIT50 cut-off) or when hHb levels are equal or above 20 µg hHb/g of feces (or equal or higher than 100ng of hemoglobin per mL; FIT100 cut-off). The FIT100 cut-off is the one commonly used in Catalonia and the rest of Spain as the cut-off value of FIT in CRC screening. As shown in Table 5, an improvement in specificity is obtained in the methods of the invention when using both thresholds, with respect to a corresponding FIT test only. In preferred embodiments, a FIT positive subject has hHb levels equal or above 10 µg hHb/g of feces (FIT50 cut-off) for which a slightly higher sensitivity is obtained.

As above mentioned, the methods of the invention may comprise in step b) quantifying any of GMLL, PTST, or BCTF, as well as any combination thereof, including i) GMLL and PTST; ii) GMLL and BCTF; iii) PTST and BCTF; and/or iv) GMLL, PTST and BCTF. Optionally, said method as defined in any of these embodiments may further comprise quantifying *Bacteroides thetaiotaomicron* (BCTT) in said intestinal sample (preferably, a feces sample). The reference strain of BCTT is *Bacteroides thetaiotaomicron* VPI-5482 strain (Bacteria; Bacteroidetes; Bacteroidia; Bacteroidales; Bacteroidaceae; Bacteroides) and its genomic sequence has GenBank access number: AE015928.1. In particular embodiments, oligonucleotides specific to SEQ ID NO: 4 may be used for the quantification of BCTT. SEQ ID NO: 4 corresponds to GenBank access number: AE015928.1 REGION: 326008..326649. In preferred embodiments, these specific oligonucleotides are any of those recited in Table 3 for BCTT, and sequences with at least 80% identity to any thereof.

1 atggcagtac aatttgaatt atataagact ccgatgccaa aggagaaaaa gaacaagacg
61 cgttatcatg cccgtccggt aagttttgag accgtcaata ccgagaaact ggcttatcgc
121 atccatgatc gctccacatt aagagtatcg gacattattt caaccttgga agaactgaaa
181 aatgaagtag ctcagtgcct cctggaaggt aaaaaggtgc atgtcgatgg attaggattc
241 tttcaggtca ctctttcctg cgaagaagaa atacgtaacc cgaaagacaa acgtgtgcac
301 cgggtaaaat tgaaagctat aaagtttaaa gctgataaag aattgaaagg ggaattgtgt
361 cacatgaaat tccagcgttc taaaatcaga ccgcactccg ccaatttatc agaagtagaa
421 atagacatga aactaactga atattttgct gaaaatcaga ttatcacccg gaaagatttt
481 caatatctct gcggaatgac acaaatcact gcatatcgcc atatcaagag gttaatggca

541 gaaaagaaat tgcaaaataa agggacgatc tatcaaccga tatatactcc ggttccgggt
601 aattacaggg tatcggtgga tttaaaatat aaagaacaat ga

Preferably, step b) comprises or consists of quantifying PTST, BCTF and BCTT.

Molecular biology methods for measuring quantities of target nucleic acid sequences are well known in the art. These methods include but are not limited to end point PCR, competitive PCR, quantitative PCR (qPCR), PCR-pyrosequencing, PCR-ELISA, DNA microarrays, nucleic acid sequencing, such as next generation sequencing methods, in situ hybridization assays such as dot-blot or Fluorescence In Situ Hybridization assay (FISH), mass spectrometry, branched DNA (Nolte, Adv. Clin. Chem. 1998,33:201-235) and to multiplex versions of said methods (see for instance, Andoh et al., Current Pharmaceutical Design, 2009;15,2066-2073) and the next generation of any of the techniques listed and combinations thereof, all of which are within the scope of the present invention.

Diverse next-generation sequencing methods have been described and are well known to a person skilled in the art. These include for instance sequencing by synthesis with cyclic reversible termination approaches (e.g., Illumina, SEQLL, Qiagen), sequencing by synthesis with single-nucleotide addition approaches (e.g., Roche-454, Thermo Fisher-Ion Torrent), sequencing by ligation (e.g., Thermo Fisher SOLiD and BGI-Complete Genomics), real-time long-read sequencing (e.g., Pacific Biosciences, Oxford Nanopore Technologies), synthetic long-read sequencing (e.g., Illumina, 10X Genomics, iGenomeX), see for instance Goodwin S, et al., Nat Rev Genet. 2016, 17(6):333-51).

In some embodiments, said molecular biology quantification methods are based on sequence specific amplification. Such an amplification-based assay comprises an amplification step which comprises contacting a sample (preferably an isolated DNA sample) with two or more amplification oligonucleotides specific for a target sequence in a target nucleic acid to produce an amplified product if the target nucleic sequence is present in the sample. The amplification product will contain a cDNA sequence corresponding to the target sequence. Suitable amplification methods include for example, replicase-mediated amplification, ligase chain reaction (LCR), strand-displacement amplification (SDA), transcription mediated amplification (TMA) and polymerase chain reaction (PCR), which includes quantitative PCR.

One particularly preferred quantification method is quantitative PCR (qPCR), also known as real-time PCR. It relates to a type of PCR that amplifies and simultaneously quantifies a target DNA molecule. Its key feature is that the amplified DNA is detected as the reaction progresses in real time. Different instruments are available, such as ABI Prism 7700 SDS, GeneAmp 5700 SDS, ABI Prism 7900 HT SDS from Applied Biosystems; iCycler iQ from Bio-Rad; Smart Cycler from Cepheid; Rotor-Gene from Corbett Research; LightCycler from Roche Molecular Biochemicals, AriaMx from Agilent and Mx4000 Multiplex from Stratagene. The qPCR process enables accurate quantification of the PCR product in real-time by measuring PCR product accumulation very early in the exponential phase of the reaction, thus reducing bias in the quantification linked to the PCR amplification efficiency occurring in end-point PCR. Real-time PCR is well known in the art and is thus not described in detail herein. Technology overview and protocols for qPCR are available for instance from the above-mentioned vendors, e.g., http://www.sigmaaldrich.com/technical-documents/protocols/biology/sybr-green-qpcr.html or http://www.sigmaaldrich.com/life-science/molecular-biology/pcr/quantitative-pcr/qpcr-technical-guide.html. For a review of qPCR methods see Wong ML y Medrano JF, Biotechniques 2005, 39(1):75-85. In a particular embodiment, the quantification method is a multiplex qPCR.

Different detecting chemistries are available for qPCR. All of them can be used with the above-mentioned qPCR instruments. The term "detection chemistry" refers to a method to report amplification of specific PCR product in real-time PCR. These detecting chemistries may be classified into two main groups; the first group comprises double-stranded DNA intercalating molecules, such as SYBR Green I and EvaGreen, whereas the second includes fluorophorelabeled oligonucleotides. The latter, in turn, has been divided into three subgroups according to the type of fluorescent molecules used in the PCR reaction: (i) primer-probes (Scorpions, Amplifluor^{®}, LUX^{™}, Cyclicons, Angler^{®}); (ii) probes; hydrolysis (TaqMan, MGB-TaqMan, Snake assay) and hybridization (Hybprobe or FRET, Molecular Beacons, HyBeacon^{™}, MGB-Pleiades, MGB-Eclipse, ResonSense^{®}, Yin-Yang or displacing); and (iii) analogues of nucleic acids (PNA, LNA^{®}, ZNA^{™}, non-natural bases: Plexor^{™} primer, Tiny-Molecular Beacon) see E. Navarro eta I.,Clinica Chimica Acta, Volume 439, 15 January 2015, Pages 231-250.

Said probes may be dual-labeled oligonucleotides, such as hydrolysis probes or molecular beacons. The 5' end of the oligonucleotide is typically labelled with a fluorescent reporter molecule while the 3' end is labeled with a quencher molecule. The sequence of the probe is specific for a region of interest in the amplified target molecule. In a more preferred embodiment, said probe is a hydrolysis probe which is designed so that the length of the sequence places the 5' fluorophore and the 3' quencher in close enough proximity so as to suppress fluorescence. Several reporter molecules and quenchers for use in qPCR probes are well known in the art.

Generally, for the quantification of nucleotide sequences specific oligonucleotides, such as probes and / or primers, are used. The term "a primer and / or a probe" specifically includes "primers and / or probes". Both expressions are used interchangeably herein and encompass for example a primer; a probe; a primer and a probe; a pair of primers; and a pair of primers and a probe. Design and validation of primers and probes is well known in the art. For the design of primers and probes in quantitative real-time PCR methods, see for instance Rodriguez A et al. (Methods Mol Biol., 2015, 1275:31-56). Preferred primers and/or probes which may be used in the methods of the invention are described herein below under the kits of the invention.

Preferably, oligonucleotides useful in the methods of the invention are about 5 to about 50 nucleotides in length, about 10 to about 30 nucleotides in length, or about 20 to about 25 nucleotides in length. In certain embodiments, oligonucleotides specifically hybridizing with the target sequence are about 19 to about 21 nucleotides in length. In a particular embodiment, said oligonucleotides have been modified for detection purposes or to enhance assay performance as described herein.

These oligonucleotides may be ribonucleotides or deoxyribonucleotides. In particular embodiments, the oligonucleotides may have at least one chemical modification. For instance, suitable oligonucleotides may be comprised of one or more "conformationally constrained" or bicyclic sugar nucleoside modifications, for example, "locked nucleic acids." "Locked nucleic acids" (LNAs) are modified ribonucleotides that contain an extra bridge between the 2' and 4' carbons of the ribose sugar moiety resulting in a "locked" conformation that confers enhanced thermal stability to oligonucleotides containing the LNAs. In other embodiments, the oligonucleotides may comprise peptide nucleic acids (PNAs), which contain a peptide-based backbone rather than a sugar-phosphate backbone. Other chemical modifications that the oligonucleotides may contain include, but are not limited to, sugar modifications, such as 2'-O-alkyl (e.g. 2'-O-methyl, 2'-O-methoxyethyl), 2'-fluoro, and 4' thio modifications, and backbone modifications, such as one or more phosphorothioate, morpholino, or phosphonocarboxylate linkages. For instance, these oligonucleotides, particularly those of shorter lengths (e.g., less than 15 nucleotides) can comprise one or more affinity enhancing modifications, such as, but not limited to, LNAs, bicyclic nucleosides, phosphonoformates, 2' O-alkyl and the like. In some embodiments, the oligonucleotides may be chemically modified, for instance to improve their resistance to nuclease degradation (e.g., by end capping), to carry detection ligands (e.g., fluorescein) or to facilitate their capture onto a solid support (e.g., poly-deoxyadenosine "tails").

The term "quantification levels" might be the concentration (DNA amount per unit of volume), the DNA amount per number of cells, the cycle threshold value (Ct value) or any mathematical transformation thereof. In a preferred embodiment, the quantification of said bacterial sequences is performed by qPCR and the quantification levels are expressed as the Ct value. The Ct (cycle threshold) value is defined as the number of qPCR cycles required for the fluorescent signal to cross the threshold. Ct levels are inversely proportional to the amount of target nucleic acid in the sample (i.e., the lower the Ct level the greater the amount of target nucleic acid in the sample).

Quantification of the abundance of a target nucleic acid sequence (e.g. SEQ ID NO: 1) in a fecal sample might be absolute or relative. Relative quantification is based on one or more internal reference genes, i.e., 16S rRNA genes from reference strains, such as determination of total bacteria (Eubacteria) using universal primers and expressing the abundance of the target nucleic acid sequence relative to Eubacteria (e.g. SEQ ID NO: 1/Eubacteria ratio). Absolute quantification gives the exact number of target molecules by comparison with DNA standards.

In a particular embodiment, optionally in combination with one or more of the embodiments or features as described herein, quantification of the bacterial markers in step b) is relative to Eubacteria and is expressed as the ratio of the corresponding quantification values (e.g. ratio of the Ct values). Specific oligonucleotides which may be used for the quantification of Eubacteria are any of those recited in Table 3 for EUB, and sequences with at least 80% identity to any thereof. Alternatively, any of the following oligonucleotides, or sequences with at least 80% identity to any thereof, may also be used:
EUB2 forward (SEQ ID NO: 5): ACTCCTACGGGAGGCAGCAGT
EUB2 reverse (SEQ ID NO: 6): GTATTACCGCGGCTGCTGGCAC

In a particular embodiment of the methods of the invention, prior to the quantification of said bacterial sequences, DNA is extracted from the feces sample. Several DNA extraction methods from fecal samples are known, all these methods relying on chemical or mechanical disruption, lysis using detergents, or a combination of these approaches (Kennedy A. et al., PLoS One, 2014;9(2):e88982). Methods for extraction of bacterial DNA in fecal samples are known from instance from M Corist et al., Journal of Microbiological Methods, 2002; 50(2):131-139, Whitney D et al., Journal of Molecular Diagnostics, American Society for Investigative Pathology, 2004;6(4):386-395 and WO2003/068788.

Preferred, methods use a combination of mechanical disruption, such as high-speed bead beating extraction, chemical lysis and a final purification step, preferably using silica membrane column such as those included in the commercially available DNA extraction kits "MobioPowerSoil^{®} DNA extraction procedure" (Mo-Bio Laboratories Inc.,), FastDNA^{®} SPIN Kit for soil procedure (MP biomedicals) and NucleoSpin^{®} Soil (Macherey-Nagel Gmbh& Co. KG). The presence of PCR inhibitors in the DNA extracts from fecal samples such as bilirubins, bile salts and complex carbohydrates is one of the difficulties faced for the determination of DNA biomarkers in DNA extracts from feces (Fleckna et al., Mol Cell Probes, 2007;21(4):282-7). Preferred DNA extraction methods are those that provide fecal extracts with a low amount of PCR inhibitors, such as less than 5%, preferably less than 2%, more preferably less than 1%, even more preferably less than 0.5%, such as less than 0.25%, 0.1%, 0.05% or 0.01%.

In some embodiments, the methods of the invention further comprise:
c) calculating a combined score from the bacterial markers levels determined in b); and
d) classifying a FOBT positive subject as having or presenting an increased risk of having AN, AA and/or CRC according to the combined score obtained in c).

The combined score of **step (c)** is a value obtained according to a given mathematical algorithm, for instance, wherein the quantification values of each of the bacterial markers determined in step b) are variables of said mathematical algorithm. Preferably, said bacterial markers are PTST, BCTF and BCTT.

In particular embodiments, when calculating the combined score, this is proportional to the absolute or relative amount of any of GMLL, PTST or BCTF in the sample; and inversely proportional to the absolute or relative amount of BCTT; wherein the higher the score, the higher the likelihood of having CRC, AA and/or AN. In other words, a high score is indicative of presence of the disease.

For instance, said combined score may be calculated as the sum of the products of standardized beta coefficients obtained in a regression analysis for each marker and wherein the bacterial marker values are used as variables. In a particular embodiment, said beta coefficients are positive for markers GMLL, PTST and/or BCTF; and negative for BCTT. A positive sign is indicative that an increase in the amount of the individual marker is associated to having or a higher likelihood of having CRC, AA and/or AN and a negative sign is indicative that a decrease of the amount of the individual marker is associated to having or a higher likelihood of having CRC, AA and/or AN.

Typically, in **step d)** said method comprises comparing the combined score in the subject sample with a reference value (e.g. reference combined score); and an increase of the combined score in the subject sample with regard to said reference value (e.g. reference combined score) is indicative of AN, AA and/or CRC.

The term "reference combined score" as used herein is a reference value obtained according to a given mathematical algorithm wherein reference expression values of each of the bacterial markers used in the method of the invention are variables of said mathematical algorithm.

The term "reference value", as used herein, relates to a predetermined criterion used as a reference for evaluating the values or data obtained from the samples collected from a subject. This "reference value" may also be referred as "cut-off value" or "threshold value".

The reference value or reference level can be an absolute value, a relative value, a value that has an upper or a lower limit, a range of values, an average value, a median value, a mean value, a z-score value (e.g. mean value + or - 1SD), a tertile value, or a value as compared to a particular control or baseline value. In a particular embodiment, optionally in combination with one or more of the embodiments or features described above or below, said reference value is the mean value or the tertile value.

In addition, it is further noted that a variety of statistical and mathematical methods for establishing the threshold or cut-off level of expression are known in the prior art. A threshold or cut-off expression level for a particular biomarker may be selected, for example, based on data from Receiver Operating Characteristic (ROC) plots. One of skill in the art will appreciate that these threshold or cut-off expression levels can be varied, for example, by moving along the ROC plot for a particular biomarker or combinations thereof, to obtain different values for sensitivity or specificity thereby affecting overall assay performance.

A reference value can be based on an individual sample value but is generally based on a large number of samples, including or excluding the sample to be tested. For instance, this reference value may be derived from one of the screening populations as defined herein above (e.g., asymptomatic adults of the general population, asymptomatic adults of over 50 years of age or asymptomatic adults having one or more risk factors) or from a population of individuals having CRC-related symptoms. Also, this reference value may be derived from a collection of tissue samples from a reference AN, AA and/or CRC patients' population for whom historical information relating to the actual clinical outcome for the corresponding cancer patient is available.

Alternatively, the reference value according to the methods of the invention can be obtained from one or more subjects not having CRC or advanced adenoma, not having a gastrointestinal disease (i.e., healthy control subjects), from subjects suffering from advanced adenoma, from subjects suffering from CRC at early stage, such as non-symptomatic (preclinical stage) or from the same subject that was diagnosed as having CRC and/or advanced adenoma but at an earlier time point.

In a particular embodiment, the method of the invention is a method of screening or diagnosis of AN, AA and/or CRC and the reference value is obtained from subjects not having AN, AA and/or CRC, or from subjects not having a gastrointestinal disease. In another embodiment, the method of the invention is a method of screening and said reference value is obtained from a screening population as defined herein. In a further embodiment, the method of the invention is a method of diagnosis and said reference value is obtained from individuals having CRC-related symptoms.

In still a further particular embodiment, the method of the invention is a method of screening or diagnosis of AN, AA and/or CRC and the reference value is obtained from subjects suffering from non-advanced adenoma. This may be the case for instance when differential diagnosis between AN, AA and/or CRC against non-advanced adenoma (NAA) is desired.

In an additional particular embodiment, the method of the invention is a method of monitoring AN, AA and/or CRC and the reference value is obtained from the same subject that was diagnosed as having AN, AA and/or CRC but at an earlier time point.

In other embodiments, the methods of the invention may comprise steps a) and b) as described herein above and further comprise:
e) comparing the amount of any of GMLL, PTST, BCTF and/or BCTT in the feces sample with corresponding reference values;
f)wherein increased amount of GMLL, PTST and/or BCTF; and decreased amount of BCTT in the sample of a FOBT positive subject with regard to the corresponding reference value is indicative of AN, AA and/ or CRC.

Preferably, wherein increased amount of PTST and BCTF and decreased amount of BCTT in the sample of a FOBT positive subject with regard to the corresponding reference value is indicative of AN, AA and/or CRC.

In the methods of the invention, the combined score (or bacterial marker amount) is considered "decreased" when said combined score (or bacterial marker amount) is lower than a reference combined score (or a reference value). Preferably, the combined score is considered to be lower than a reference combined score (or a reference value) when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than the reference combined score (or a reference value).

Likewise, in the context of the methods of the invention, the combined score (or bacterial marker amount) is considered "increased" when said combined score is higher than a reference combined score (or a reference value). Preferably, the combined score (or bacterial marker amount) is considered to be higher than a reference combined score (or a reference value) when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than a reference combined score (or a reference value).

Alternatively or in addition, subjects having more than about 1.1, 1.2, 1.3, 1.4, 1.5, 2, 3, 4, 5, 6, 7, 8, 9, 10, 15 or 20 fold levels deviation (i.e., increase or decrease) than the reference combined score (or reference value) as described herein.

The methods of the invention, as it is understood by a person skilled in the art, do not claim to be correct in 100% of the analyzed samples. However, it requires that a statistically significant amount of the analyzed samples is classified correctly. The amount that is statistically significant can be established by a person skilled in the art by means of using different statistical significance measures obtained by statistical tests, illustrative, non-limiting examples of said statistical significance measures include determining confidence intervals, determining the p-value, etc. Preferred confidence intervals are at least 90%, at least 95%, at least 97%, at least 98%, at least 99%. The p-values are, preferably less than 0.1, less than 0.05, less than 0.01, less than 0.005 or less than 0.0001. The teachings of the present invention preferably allow correctly classifying at least 60%, at least 70%, at least 80%, or at least 90% of the subjects of a determining group or population analyzed.

Validity studies address the agreement between a proposed (index) test and a reference standard for the ability to identify a target condition (see Florkowski M. C., Clin Biochem Rev. 2008, 29 (Suppl 1): S83-S87). Sensitivity, specificity, accuracy, positive likelihood ratio, negative likelihood ratio, positive predictive value and negative predictive value are statistic values which can be defined to evaluate the test performance. Acronyms' definition and further details are provided in Table 1 below.

| | Reference Standard | | |
|---|---|---|---|
| | Disease present | Disease absent | Total |
| Index Test positive | True positive (TP) | False positive (FP) | TP + FP |
| Index Test negative | False negative (FN) | True negative (TN) | TN + FN |
| Total | TP + FN | TN + FP | |
| Sensitivity = TP / (TP + FN) | | | |
| Specificity = TN / (TN + FP) | | | |
| Positive predictive value (PPV) = TP / (TP + FP) | | | |
| Negative predictive value (NPV) = TN / (TN + FN) | | | |
| Positive likelihood ratio (LR+) = sensitivity / (1 - specificity) | | | |
| Negative likelihood ratio (LR-) = (1 - sensitivity) / specificity | | | |

A test is usually calibrated in terms of the desired specificity and sensitivity according to the target use of the test in clinical practice. High sensitivity corresponds to high negative predictive value and is considered generally a desired property for a "rule out" test, such as a screening test which typically will be followed by a confirmatory test. High specificity corresponds to high positive predictive value and is considered generally a desired property for a "rule in" test, such as a companion diagnostic test.

In preferred embodiments, the methods of the invention have sensitivity, specificity and/or accuracy values of at least about 60%, preferably of at least about 70%, and can be, for example, at least 75%, at least 80%, at least 85%, at least 90%, at least 95%, at least 96%, at least 97%, at least 98%, at least 99% or 100% in at least 60% of the group or population assayed, or preferably in at least 65%, 70%, 75%, 80%, 85%, 90%, 95% or 100% of the group or population assayed.

It is further noted that the accuracy of the method of the invention can be further increased by additionally considering other gene or bacterial markers, biochemical parameters and/or clinical characteristics of the patients (e.g. age, sex, tobacco and/or other risk factors). Determination of these other markers, parameters and/or characteristics can be sequential or simultaneous to any or all of steps of the methods of the invention as described herein.

In a particular embodiment, optionally in combination with one or more of the features or embodiments described herein, the methods of the invention further comprise quantifying one or more bacterial markers selected from the group consisting of B10, B46, B48, *Roseburia intestinalis* (RSBI), *Collinsella intestinalis* (CINT), *Faecalibacterium prausnitzii* (Duncan et al., Int. J. Syst. Evol. Microbiol 2002, 52:2141-2146) and/or any of its phylogroups (see; WO2017/025617 A1 and Lopez-Siles et al. Appl Environ Microbiol. 2012, 78:420-428), and *Escherichia coli.*

B10 has sequence SEQ ID NO: 7 (shown below) corresponding to an uncultured bacterium isolate DGGE gel band Eub_10 16S ribosomal RNA gene, partial sequence (GenBank: GQ411118.1). In particular embodiments, oligonucleotides specific to SEQ ID NO: 7 may be used for the quantification of B10.

1 cttcggattg taaactcctg ttgttgagga gataatgacg gtactcaaca aggtaagtga
61 cggctaacta cgtgccagca gccgcggtaa aacgtaggtc acaagcgttg tccggaatta
121 ctgggtgtaa agggagcgca ggcgggaaga caagttggaa gtgaaatcca tgggctcaac
181 ccatgaactg ctttcaaaac tgtttttctt gagtagtgca caggtaggcg gaattcccgg
241 tgtagcggtg gaatgcgtag atatcgggag gaacaccagt ggcgaaggcg gcctactggg
301 caccaactga cgctgaggct cgaaagtgtg ggtagcaaac aggattagat accctggtag
361 tccacactgt aaacgatgat tactaggtgt tggaggattg accccttcag tgccgcagtt
421 aacacaataa gtaatccacc tggggagtac gaccgcaagg ttgaaactca aaggaattga
481 cggac

B46 has sequence SEQ ID NO: 8 (shown below) corresponding to an uncultured bacterium isolate DGGE gel band Eub_46 16S ribosomal RNA gene, partial sequence (GenBank: GQ411150.1). In particular embodiments, oligonucleotides specific to SEQ ID NO: 8 may be used for the quantification of B46.

1 gtaacatttc tgtaacaaga catacagcag tttctcagag ttcccaaact caaaggaatt
61 gacggaagcc gcggttccac gtaagtcaca agcgttgtcc ggaattactg ggtgtaaagg
121 gagcgcaggc gggaagacaa gttggaagtg aaatccatgg gctcaaccca taaactgctt
181 tcctaactgt ttttcttgag tagtgcacag gtaggcggaa ttcccggtgt agcggtggaa
241 tgcctagata tcgggaggaa caccattggc gaaggcggcc tactggactg caactgacgc
301 tgaggctcga aagtgtgggt agcaaacagg attagatacc ctggtagtcc actccgtaaa
361 ccatgattac tacgtgttgg aggattgacc ccttctgtgc cgcagataac acaataagta
421 atccacctgg ggagtacgac cgcccggttg agactcacag gaattgacgg actc

B48 has sequence SEQ ID NO: 9 (shown below) corresponding to an uncultured bacterium isolate DGGE gel band Eub_48 16S ribosomal RNA gene, partial sequence (GenBank: GQ411152.1). In particular embodiments, oligonucleotides specific to SEQ ID NO: 9 may be used for the quantification of B48.

1 tctcttacgg ggagcagcag tggggaatat tgcacaatgg gggaaaccct gatgcagcga
61 cgccgcgtga gcgatgaagt atttcggtat gtaaagctct atcagcaggg aagaaaatga
121 cggtacctga ctaagaagcc ccggctaaat acgtgccagc agccgcggta atacgtatgg
181 tgcaagcgtt atccggattt actgggtgta aagggagcgt agacggagtg gcaagtctga
241 tgtgaaaacc cggggctcaa ccccgggact gcattggaaa ctgtgcatct agagtgtcgg
301 agaggtaagc ggaattccta gtgtagcggt gaaatgcgta gatattagga ggaacaccag
361 tggcgaaggc ggcttactgg acgataactg acgctgaggc tcgaaagcgt ggggagcaaa
421 caggattaga taccctggta gtccacgccg taaacgatga ctactaggtg tcggggagca
481 cagctcttcg gtgccgcagc aaacgcaata agtattccac ctggggagta cgttcgcaag
541 aatgaaactc acagaatttg acggag

The reference strain of RSBI is *Roseburia intestinalis* L1-82 strain (Bacteria; Firmicutes; Clostridia; Clostridiales; Lachnospiraceae; Roseburia) and its genomic sequence is shown in GenBank access number: LR027880.1. In particular embodiments, oligonucleotides specific to SEQ ID NO: 10 may be used for the quantification of RSBI. SEQ ID NO: 10 corresponds to GenBank access number LR027880.1 REGION: 599208..600101.

1 atgtcttttt caagtgaagt gaaacaggag ttagcaaagc agagtggaaa gagcagacat
61 tgtcagatcg ctgaacttgc ggcgttagtt gcttttgacg gaaagcgtca gcagctgatc
121 ggggatgcgg gagatgtgct ggattcggaa aatccgcttc tgcaggaaaa atatggactg
181 ttactggcac agctgttcca tgttaatata gaagagatag atacattgcc accggaacgc
241 attcttgaga caatcaagat gtggaatcag tctttaaggt gtgcagatat cacagagacg
301 gtaaatggta ttttactgca gcagacatgt tgcaggcggg catatatccg ggagcattt
361 ttagcaggtg gatcgatcag tgatccgaat aagtcttatc attttgagat tgtctgccgg
421 gaaattgcgc aggcgaaaca gcttcaggat gcaatcaaca gttttgagat ggaagcaaag
481 atcgtagaac gaaagaaaca ccaggtcgta tatttaaagg aaggtgccca gatcgtggat
541 atgttaaata tcatggaagc acatgtggca ctgatgaatc ttgaaaatgt gcgtatctta
601 aaagaaatga gaaattctgt caaccgaaag gtaaattgtg agacggcaaa tatcagcaag
661 actgtggcgg cagccgtaaa acagcttggc gatattgaat atattaaaca gaatgcaggc
721 ttagacagtc tgcctgaaaa cctaaaagaa atggcgttgc tgcggttaga gtatccggat
781 acaccgctta aggaactggg aacatatctc gatcctccgg tcggaaaatc gggagtgaac
841 cacaggctgc gcaggatcag tgagattgca gatgagctgc gtgaaaaaga ataa

The reference strain of CINT (Bacteria; Actinobacteria; Coriobacteriia; Coriobacteriales; Coriobacteriaceae; Collinsella) is *Collinsella intestinalis* DSM 13280 strain and its genomic sequence has NCBI Reference Sequence: GG692711.1. In particular embodiments, oligonucleotides specific to SEQ ID NO: 11 may be used for the quantification of CINT. SEQ ID NO: 11 corresponds to NCBI Reference Sequence: GG692711.1 REGION: 297314..298120.

1 atgtccggac actctaaatg ggcaactacc aagcaccgca agggtgcgca ggacgctaag
61 cgttccgccc tgttctccaa gctgagccgt aacatcaccg tcgcggcccg tcttggcaac
121 gaccccaacc cggacaacaa cgcctccctc gccgctgccg tggccaaggc caaggctcag
181 tccatgccca aggacaagat caaggccgcc atcgacaagg ccttcggttc cggcgccgac

241 gccgccgtgt acgagaacat cgtgtacgag ggctacggcc cggccggtgt tgccgtgtac
301 gtcgagtgcc tgaccgacaa ccgcaaccgc accgccgccg atgtccgctc cgccttctcc
361 cacgctggcg gcaacctggg caccaccggc tccgttgcct tccagttcga gcgcaagggc
421 caggtcgtgg tctccaagca gatcgtcgac ccgaacgaca agaaggagaa cctcatggcc
481 aacggcgctg ccggcgatga ggaagagttc atgatggtca tcgccgaggc cggtggcgac
541 gactacgagg acgccggtga cgagtgggtc gtgtggaccg ccgcgggcga cctcatggcc
601 gtgtccaagg gcatcgaggc tcagggcatc gaggtcaagg gtgccgagct caccatggtc
661 ccgaccaccc cgacggccgt ctccggcgcc gacgccaaga aggtccagcg cctcatcgac
721 cgcctcgagg acctggacga cgtccaggac gtgtactcca ccatggacat gaccgacgag
781 gtcatcgctg ccctcgaaga ggagtaa

The reference strain of *Faecalibacterium prausnitzii* (Bacteria; Firmicutes; Clostridia; Clostridiales; Ruminococcaceae; faecalibacterium) *is Faecalibacterium prausnitzii A2-165* (GenBank access number: AJ270469.2), which is also the representative strain of its Phylogroup II (PHGII). *Faecalibacterium prausnitzii* M21/2 (GenBank access number: DS483503.1) is the representative strain of its Phylogroup I (PHGI). In particular embodiments, oligonucleotides specific to SEQ ID NO: 12 may be used for the quantification of *Faecalibacterium prausnitzii,* SEQ ID NO: 12 (shown below) corresponds to GenBank access number: AJ270469.2.

1 gttgatcctg gctcaggacg aacgctggcg gcgcgcctaa cacatgcaag tcgaacgagc
61 gagagagagc ttgctttctc gagcgagtgg cgaacgggtg agtaacgcgt gaggaacctg
121 cctcaaagag ggggacaaca gttggaaacg actgctaata ccgcataagc ccacagctcg
181 gcatcgagca gagggaaaag gagcaatccg ctttgagatg gcctcgcgtc cgattagcta
241 gttggtgagg taatggccca ccaaggcaac gatcggtagc cggactgaga ggttgaacgg
301 ccacattggg actgagacac ggcccagact cctacgggag gcagcagtgg ggaatattgc
361 acaatggggg aaaccctgat gcagcgacgc cgcgtggagg aagaaggtct tcggattgta
421 aactcctgtt gttgaggaag ataatgacgg tactcaacaa ggaagtgacg gctaactacg
481 tgccagcagc cgcggtaaaa cgtaggtcac aagcgttgtc cggaattact gggtgtaaag
541 ggagcgcagg cgggaagaca agttggaagt gaaatctatg ggctcaaccc ataaactgct
601 ttcaaaactg tttttcttga gtagtgcaga ggtaggcgga attcccggtg tagcggtgga
661 atgcgtagat atcgggagga acaccagtgg cgaaggcggc ctactgggca ccaactgacg
721 ctgaggctcg aaagtgtggg tagcaaacag gattagatac cctggtagtc cacaccgtaa
781 acgatgatta ctaggtgttg gaggattgac cccttcagtg ccgcagttaa cacaataagt
841 aatccacctg gggagtacga ccgcaaggtt gaaactcaaa ggaattgacg ggggcccgca
901 caagcagtgg agtatgtggt ttaattcgac gcaacgcgaa gaaccttacc aagtcttgac
961 atcctgcgac gatgctggaa acagtatttt ccttcgggac gcagagacag gtggtgcatg
1021 gttgtcgtca gctcgtgtcg tgagatgttg ggttaagtcc cgcaacgagc gcaaccctta
1081 ctgtcagtta ctacgcaaga ggactctggc aggactgccg ttgacaaaac ggaggaaggt
1141 ggggatgacg tcaaatcatc atgcccttta tgacttgggc tacacacgta ctacaatggc
1201 gttaaacaaa gagaagcaag accgcgaggt ggagcaaaac tcagaaacaa cgtcccagtt
1261 cggactgcag gctgcaactc gcctgcacga agtcggaatt gctagtaatc gtggatcagc
1321 atgccacggt gaatacgttc ccgggccttg tacacaccgc ccgtcacacc atgagagccg
1381 gggggacccg aagtcggtag tctaaccgca aggaggacgc cgccgaaggt aaaactggtg
1441 attggggtga agtcgtaaca aggtac

The reference strain of *Escherichia coli* (Bacteria; Proteobacteria; Gammaproteobacteria; Enterobacterales; Enterobacteriaceae; Escherichia) is *Escherichia coli* str. K-12 substr. MG1655 and its genomic sequence has GenBank access number: CP032667.1. In particular embodiments, oligonucleotides specific to SEQ ID NO: 13 (shown below) may be used for the quantification of *Escherichia coli.*

1 cgccctcccg aaggttaagc tacctacttc ttttgcaacc cactcccatg gtgtgacggg
61 cggtgtgtac aaggcccggg aacgtattca ccgtggcatt ctgatccacg attactagcg
121 attccgactt catggagtcg agttgcagac tccaatccgg actacgacgc actttatgag
181 gtccgcttgc tctcgcgagg tcgcttctct ttgtatgcgc cattgtagca cgtgtgtagc
241 cctggtcgta agggccatga tgacttgacg tcatccccac cttcctccag tttatcactg
301 gcagtctcct ttgagttccc ggccggaccg ctggcaacaa aggataaggg ttgcgctcgt
361 tgcgggactt aacccaacat ttcacaacac gagctgacga cagccatgca gcacctgtct
421 cacggttccc gaaggcacat tctcatctct gaaaacttcc gtggatgtca agaccaggta
481 aggttcttcg cgttgcatcg aattaaacca catgctccac cgcttgtgcg ggcccccgtc
541 aattcatttg agttttaacc ttgcggccgt actccccagg cggtcgactt aacgcgttag
601 ctccggaagc cacgcctcaa gggcacaacc tccaagtcga catcgtttac ggcgtggact
661 accagggtat ctaatcctgt ttgctcccca cgctttcgca cctgagcgtc agtcttcgtc
721 cagggggccg ccttcgccac cggtattcct ccagatctct acgcatttca ccgctacacc
781 tggaattcta cccccctcta cgagactcaa gcttgccagt atcagatgca gttcccaggt
841 tgagcccggg gatttcacat ctgacttaac aaaccgcctg cgtgcgcttt acgcccagta
901 attccgatta acgcttgcac cctccgtatt accgcggctg ctggcacgga gttagccggt
961 gcttcttctg cgggtaacgt caatgagcaa aggtattaac tttactccct tcctccccgc
1021 tgaaagtact ttacaacccg aaggccttct tcatacacgc ggcatggctg catcaggctt
1081 gcgcccattg tgcaatattc cccactgctg cctcccgtag gagtctggac cgtgtctcag
1141 ttccagtgtg gctggtcatc ctctcagacc agctagggat cgtcgcctag gtgagccgtt
1201 accccaccta ctagctaatc ccatctgggc acatccgatg gcaagaggcc cgaaggtccc
1261 cctctttggt cttgcgacgt tatgcggtat tagctaccgt ttccagtagt tatccccctc
1321 catcaggcag tttcccagac attactcacc cgtccgccac tcgtcagcaa agaagcaagc
1381 ttcttcctgt taccgttcga cttgcatgtg ttaggcctgc cgccagcgtt caatctgagc
1441 catgatcaaa ct

In preferred embodiments, any of the specific oligonucleotides recited in Table 3, and sequences with at least 80% identity to any thereof, can be used for the quantification of B10, B46, B48, CINT, or RSBI, respectively. Also, in preferred embodiments, any of the specific oligonucleotides recited in Table 2 of WO2017/025617 A1, and sequences with at least 80% identity to any thereof, can be used for the quantification of *Faecalibacterium prausnitzii,* its phylogroups (i.e., PHGI and PHGII) and *Escherichia coli.*

The methods of the present invention or any of the steps thereof might be implemented by a computer. The term computer as used herein may refer to any programmable apparatus or system. In a particular embodiment, optionally in combination with any of the features or embodiments as described herein, the combined score of step c) is calculated using a computer; and/or the selection, classification and/or determination of step d) is conducted using a computer. Therefore, a further aspect of the invention refers to a computer implemented method wherein the combined score of step
c) is calculated using a computer; and/or the selection, classification and/or determination of step d) is conducted using a computer.

It is noted that any computer program capable of implementing any of the methods of the present invention or used to implement any of these methods or any combination thereof, also forms part of the present invention.

This computer program is typically directly loadable into the internal memory of a digital computer, comprising software code portions for performing the steps of comparing the combined score (e.g., obtained from the level of one or more of the target markers as described herein), from the one or more biological samples of a subject with a reference value (e.g., reference combined value) and determining the diagnosis of said subject when said product is run on a computer.

It is also noted that any device or apparatus comprising means for carrying out the steps of any of the methods of the present invention or any combination thereof, or carrying a computer program capable of, or for implementing any of the methods of the present invention or any combination thereof, is included as forming part of the present specification.

The methods of the invention may also comprise the storing of the method results in a data carrier, preferably wherein said data carrier is a computer readable medium. The present invention further relates to a computer-readable storage medium having stored thereon a computer program of the invention or the results of any of the methods of the invention.

As used herein, "a computer readable medium" can be any apparatus that may include, store, communicate, propagate, or transport the results of the determination of the method of the invention. The medium can be an electronic, magnetic, optical, electromagnetic, infrared, or semiconductor system (or apparatus or device) or a propagation medium.

### Methods for treatment and related medical uses

In a further aspect, the present invention provides a method of treating a subject having AN, AA and/or CRC, wherein said subject has been selected by a method for the screening, diagnosis and/or monitoring as described herein above, and wherein said method further comprises administering to the subject an anti-cancer therapy.

In a related aspect, the present invention also provides any of the methods as described herein above, which further comprises a step of administering to said patient a therapeutically effective amount of an anti-cancer therapy.

In another aspect, the invention relates to an anti-cancer therapy for use in a method for treatment of a cancer patient, wherein said cancer patient has been selected by a screening, diagnostic and/or monitoring method as described herein.

In an additional aspect, the invention also refers to a method for selecting a subject for conducting an exploratory test selected from the group consisting of colonoscopy, flexible sigmoidoscopy, double-contrast barium enema and computed tomography (CT) colonography, preferably for conducting a colonoscopy, wherein said subject has been selected by a screening, diagnosis or monitoring method as described herein.

In still a further aspect, the invention pertains to a method for conducting an exploratory test in a subject, wherein said subject has been selected by a screening, diagnosis or monitoring method as described herein, wherein said method further comprises conducting an exploratory test to the subject, wherein the test is selected from the group consisting of colonoscopy, flexible sigmoidoscopy, double-contrast barium enema and computed tomography (CT) colonography, preferably a colonoscopy.

### Kit and use of a kit in the methods of the invention

In another aspect, the invention provides a kit suitable for quantifying any of GMLL, PTST, BCTF or BCTT, wherein said kit comprises one or more of the following reagents:
i. an oligonucleotide specific to GMLL genome, preferably an oligonucleotide specific to SEQ ID NO:1 ;
ii. an oligonucleotide specific to PTST genome, preferably an oligonucleotide specific to SEQ ID NO:2;
iii. an oligonucleotide specific to BCTF genome, preferably an oligonucleotide specific to SEQ ID NO:3 ;
iv. an oligonucleotide specific to BCTT genome, preferably an oligonucleotide specific to SEQ ID NO:4; and
v. optionally, an oligonucleotide suitable for the quantification of Eubacteria as defined herein above.

Optionally, further comprising instructions for the use of said reagents in determining said bacterial markers levels in a feces sample isolated from a subject.

The term "kit" or "testing kit" denotes combinations of reagents and adjuvants required for an analysis. Although a test kit consists in most cases of several units, one-piece analysis elements are also available, which must likewise be regarded as testing kits.

In preferred embodiments, said kit is suitable for quantifying PTST, BCTF and BCTT, and said kit comprises reagents ii), iii) and iv) as defined herein above. Preferably, it further comprises reagent v) as defined above.

In particular embodiments, optionally in combination with one or more of the features or embodiments as described herein,
- the oligonucleotide specific to SEQ ID NO:1 is an oligonucleotide comprising or consisting of any of SEQ ID NO:22, or SEQ ID NO:23, or a sequence with at least 80% identity to any thereto; and/or
- the oligonucleotide specific to SEQ ID NO:2 is an oligonucleotide comprising or consisting of any of SEQ ID NO:24, or SEQ ID NO:25, or a sequence with at least 80% identity to any thereto; and/or
- the oligonucleotide specific to SEQ ID NO:3 is an oligonucleotide comprising or consisting of any of SEQ ID NO:26, or SEQ ID NO:27, or a sequence with at least 80% identity to any thereto; and/or
- the oligonucleotide specific to SEQ ID NO:4 is an oligonucleotide comprising or consisting of any of SEQ ID NO:30, or SEQ ID NO:31, or a sequence with at least 80% identity to any thereto; and/or
- the oligonucleotide suitable for the quantification of Eubacteria is an oligonucleotide comprising or consisting of any of SEQ ID NO: 5, SEQ ID NO: 6, SEQ ID NO:14, or SEQ ID NO:15, or a sequence with at least 80% identity to any thereto.

Preferred embodiments relate to a kit suitable for quantifying PTST, BCTF and BCTT, wherein said kit comprises the following reagents:
- an oligonucleotide comprising or consisting of any of SEQ ID NO:24, or SEQ ID NO:25, or a sequence with at least 80% identity to any thereto;
- an oligonucleotide comprising or consisting of any of SEQ ID NO:26, or SEQ ID NO:27, or a sequence with at least 80% identity to any thereto; and
- an oligonucleotide comprising or consisting of any of SEQ ID NO:30, or SEQ ID NO:31, or a sequence with at least 80% identity to any thereto; and
- optionally, an oligonucleotide suitable for the quantification of Eubacteria (preferably an oligonucleotide comprising or consisting of any of SEQ ID NO: 5, SEQ ID NO: 6, SEQ ID NO:14, or SEQ ID NO:15, or a sequence with at least 80% identity to any thereto);
- optionally, further comprising instructions for the use of said reagents in determining said bacterial markers levels in a feces sample isolated from a subject.

Said specific oligonucleotides are as described herein above. Preferably, said oligonucleotide is a primer and/or probe.

Further preferred embodiments relate to a kit suitable for quantifying PTST, BCTF and BCTT, wherein said kit comprises the following reagents:
- a primer comprising or consisting of SEQ ID NO:24, a primer comprising or consisting of SEQ ID NO:25, or a sequence with at least 80% identity to any thereto;
- a primer comprising or consisting of SEQ ID NO:26, a primer comprising or consisting of SEQ ID NO:27, or a sequence with at least 80% identity to any thereto; and
- a primer comprising or consisting of SEQ ID NO:30, a primer comprising or consisting of SEQ ID NO:31, or a sequence with at least 80% identity to any thereto; and
- optionally, an oligonucleotide suitable for the quantification of Eubacteria (preferably a primer comprising or consisting of SEQ ID NO: 5, a primer comprising or consisting of SEQ ID NO: 6, a primer comprising or consisting of SEQ ID NO:14, or a primer comprising or consisting of SEQ ID NO:15, or a sequence with at least 80% identity to any thereto);
- optionally, further comprising instructions for the use of said reagents in determining said bacterial markers levels in a feces sample isolated from a subject.

In preferred embodiments, said sequence with at least 80% identity is a sequence at least about 85%, preferably at least about 90%, more preferably at least about 95%, 96%, 97%, 98%, or 99% identical to the reference sequence. Identity percentage between the two sequences can be determined by any means known in the art, for example the Needleman and Wunsch global alignment algorithm.

In addition, said kit may further comprise oligonucleotides specific for other gene or bacterial markers as described herein above. For instance, wherein said other bacterial markers are selected from the group consisting of B10, B46, B48, CINT, RSBI, *Faecalibacterium prausnitzii, F. prausnitzii* PHGI, *F. prausnitzii* PHGII and *E. coli.*

Said kit may comprise additional reagents. In a particular embodiment, said kit comprises reagents to perform a real-time PCR reaction, which typically contain a DNA polymerase, such as Taq DNA polymerase {e.g., hot- start Taq DNA polymerase), buffer, magnesium, dNTPs, and optionally other agents (e.g., stabilizing agents such as gelatin and bovine serum albumin). In addition, real-time PCR reaction mixtures also contain reagents for real time detection and quantification of amplification products as described above herein.

Optionally, the kit can also include appropriate tubes and solvents for DNA extraction, e.g., a chloroform/methanol solution. Moreover, the kit may also comprise a recipient for collecting the feces sample and optionally, any buffer and/or additives adequate for the sample preservation, for instance the FIT tube collector used in the examples. Other preferred features and embodiments of the kit of the invention are as described herein throughout the specification.

In a further aspect, the present invention also provides a kit as described herein for use in a method for the screening, diagnosis and/or monitoring of AN, AA and/or CRC as described herein.

It is contemplated that any features described herein can optionally be combined with any of the embodiments of any method, medical use, kit and use of a kit of the invention; and any embodiment discussed in this specification can be implemented with respect to any of these. It will be understood that particular embodiments described herein are shown by way of illustration and not as limitations of the invention.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

The following examples serve to illustrate the present invention and should not be construed as limiting the scope thereof.

### EXAMPLES

### Example 1.- Material and Methods

### Study population

A cohort consisting of 333 consecutive patients with CRC-related symptoms referred for a diagnostic colonoscopy from primary and secondary health care to Complexo Hospitalario de Ourense (Ourense, Spain) was recruited (Table 1). Exclusion criteria were: (1) asymptomatic subjects undergoing colonoscopy for CRC screening, (2) patients with a previous history of colonic disease undergoing surveillance colonoscopy, (3) patients requiring hospital admission, (4) patients whose symptoms had ceased within 3 months before evaluation, and (5) patients who had received antibiotic treatment within the last month prior to inclusion. The study protocol was approved by the Biobanco del Complexo Hospitalario Universitario de Vigo (Vigo, Spain). Written informed consent was obtained from all study patients.

**Table 1. Patients characteristics classified according to colonoscopy diagnostic. Hb, hemoglobin; FIT100 (20 µg Hb/g of feces); CRC, colorectal cancer; AA, advanced adenoma; NAA, non-advanced adenoma; NC, normal colonoscopy.**

| **Characteristics** | **CRC** | **AA** | **NAA** | **NC** |
|---|---|---|---|---|
| **n (%)** | 48 (14.4) | 30 (9) | 88 (26.4) | 167 (50.2) |
| **Age (mean, range)** | 73 (53-91) | 65 (44-83) | 67 (37-89) | 61 (20-87) |
| **Sex, female (%)** | 17 (10) | 15 (8.8) | 32 (18.8) | 106 (62.4) |
| **FIT100 (%)** | 47 (97.9) | 18 (60) | 30 (34.1) | 21 (12.6) |

All subjects underwent colonoscopy in order to determine their colorectal status. According to the endoscopic examination and the pathology results, diagnosis was classified into four groups: normal colonoscopy (colonoscopy with no findings or with sigma and/or rectum hyperplastic polyps <10 mm), non-advanced adenomas (tubular adenomas <10 mm with low grade dysplasia, and serrated polyps <10 mm without dysplasia), advanced adenomas (adenomas >10 mm or with villous component or high grade dysplasia, serrated polyps >10 mm or with dysplasia, and pTis adenocarcinoma) and invasive CRC. Patients diagnosed with CRC were also classified according to the stage of the tumor (Table 2). Individuals were also asked to answer a questionnaire in order to record clinical and epidemiologic data.

**Table 2. Patients with colorectal cancer according to tumor TNM stage. CRC, colorectal cancer.**

| **CRC stage** | **n (%)** |
|---|---|
| **0** | 3 |
| **I** | 6 |
| **II** | 10 |
| **III** | 21 |
| **IV** | 8 |

### Fecal sample collection

Participants were asked to collect a stool sample from one bowel movement in a sterile feces container before colonoscopy and prior to bowel cleanse. Samples were immediately frozen after deposition. Then, subjects brought samples to the hospital, where they were kept frozen at -20 °C for short-term storage and stored at -80 °C upon arrival at the GoodGut S.L. facilities in Girona (Spain). A total of 11 subjects were excluded from the study due to the wrong stool samples collection.

### DNA extraction from stool samples

Genomic DNA was extracted from frozen fecal samples after homogenization using the NucleoSpin Soil Kit (Macherey-Nagel GMbH & Co., Duren, Germany). The instructions of manufacturer were followed, DNA was finally eluted in a 100 µl final volume of SE Elution Buffer and stored at -20 °C until use. DNA concentration was determined with Qubit fluorometric quantification (ThermoFisher Scientific, Waltham, USA). All samples were adjusted to a final concentration of 8 ng/µl and quantified again.

### qPCR assay for CRC biomarkers

The specific bacterial sequences targeted were ten: Eubacteria (EUB), B10 (best match BLAST *Faecalibacterium prausnitzii*), B46 (best match BLAST *Subdoligranulum variabile),* B48 (best match BLAST *Ruminococcus* sp., *Roseburia* sp., *Coprococcus* sp.), *Roseburia intestinalis* (RSBI); *Gemella morbillorum* (GMLL), *Peptostreptococcus stomatis* (PTST), *Bacteroides fragilis* (BCTF), *Collinsella intestinalis* (CINT); and *Bacteroides thetaiotaomicron* (BCTT).

Quantification of the different biomarkers was performed by preparing single reaction for each biomarker using SYBR Green Master Mix (Promega, Madison, USA). Each reaction consisted of 20 µl containing 1× GoTaq qPCR Master Mix, between 150 nM and 300 nM of each primer, and up to 20 ng of genomic DNA template. The species-specific primers used in this study are shown in Table 3 and were purchased at Macrogen (Macrogen, Seoul, South Korea).

**Table 3. Forward and reverse primers used in this study. EUB, Eubacteria; B10, B46, B48, GMLL, Gemella morbillorum; PTST, Peptostreptococcus stomatis; BCTF, Bacteroides fragilis; CINT, Colinsella intestinalis; BCTT, Bacteroides thetaiotaomicron; RSBI, Roseburia intestinalis.**

| **Target** | **Primers** | SEQ ID NO: | **Sequence 5'** → **3**' | **Primer concentration** | **References** |
|---|---|---|---|---|---|
| EUB | EUB_F | SEQ ID NO: 14 | ACT CCT ACG GGA GGC AGC AGT | 200 nM | Modified |
| | EUB_R | SEQ ID NO:15 | GTA TTA CCG CGG CTG CTG GCA C | | (Matsuda et al, 2011) |
| B10 | B10_F | SEQ ID NO:16 | CAA CAA GGT AAG TGA CGG C | 300 nM | (Mas de Xaxars Rivero, 2012; Serra-Pagès et al, 2015) |
| | B10_R | SEQ ID NO:17 | CGC CTA CCT GTG CAC TAC TC | | |
| B46 | B46_F | SEQ ID NO:18 | TCC ACG TAA GTC ACA AGC G | 300 nM | (Mas de Xaxars Rivero, 2012; Serra-Pagès et al, 2015) |
| | B46_R | SEQ ID NO:19 | CGC CTA CCT GTG CAC TAC TC | | |
| B48 | B48_F | SEQ ID NO:20 | GTA CGG GGA GCA GCA GTG | 300 nM | (Mas de Xaxars Rivero, 2012; Serra-Pagès et al, 2015) |
| | B48_R | SEQ ID NO:21 | GAC ACT CTA GAT GCA CAG TTT CC | | |
| GMLL | GMLL_F | SEQ ID NO:22 | AAG AGT TCC AAG GCG TTC TC | 150 nM | This study |
| | GMLL_R | SEQ ID NO:23 | CCA TTT CAA GAT CCG CTT TCT ATT T | | |
| PTST | PTST_F | SEQ ID NO:24 | AGG TTG ATG CTC TGA GTA GTA G | 150 nM | This study |
| | PTST_R | SEQ ID NO:25 | ATG AAT ACT AGC CTC TCC TCT TT | | |
| BCTF | BCTF_F | SEQ ID NO:26 | TGA AAG CGT GCT CTT ACT ATT G | 150 nM | This study |
| | BCFT_R | SEQ ID NO:27 | TAT TGG CTG TTG TGC TTT GT | | |
| CINT | CINT_F | SEQ ID NO:28 | GAC CAT CAT GAA CTC TTC CTC | 150 nM | This study |
| | CINT_R | SEQ ID NO:29 | CCG TTG CCT TCC AGT TC | | |
| BCTT | BCTT_F | SEQ ID NO:30 | AGT GAC CTG AAA GAA TCC TAA T | 150 nM | This study |
| | BCTT_R | SEQ ID NO:31 | GAC CGT CAA TAC CGA GAA AC | | |
| RSBI | RSBI_F | SEQ ID NO:32 | GTG CCA GTA ACA GTC CAT ATT | 150 nM | This study |
| | RSBI_R | SEQ ID NO:33 | TAG CAA AGC AGA GTG GAA AG | | |

All quantitative PCR were run on an AriaMx Realtime PCR System (Agilent Technologies, Santa Clara, USA). Thermal profiles were different according to the biomarker analyzed (Table 4). A melting curve step was added to the end of each qPCR to verify the presence of the expected amplicon size as well as to control primer dimmer formation. Data were collected and analyzed with the Aria Software version 1.3 (Agilent Technologies, Santa Clara, USA). All samples were amplified in duplicates, which were considered valid when the difference between threshold cycles (Ct) was less than 0.6. A dynamic range consisting of 8 logs from 10 to 10⁸ genome units/µL was established for each biomarker and was used to calculate the relative abundance. A no-template control reaction was included in each PCR run.

**Table 4. qPCR conditions for EUB, Eubacteria; B10, B46, B48, GMLL, Gemella morbillorum; PTST, Peptostreptococcus stomatis; BCTF, Bacteroides fragilis; CINT, Colinsella intestinalis; BCTT, Bacteroides thetaiotaomicron; RSBI, Roseburia intestinalis.**

| **Bacterial markers** | **Total cycles** | **Denaturing** | | **Annealing and Extension** | | **Melting curve** | |
|---|---|---|---|---|---|---|---|
| | | **T^{a} (°C)** | **Time (min:s)** | **T² (°C)** | **Time (min:s)** | **T^{a} (°C)** | **Time (min:s)** |
| EUB | 40 | 95 | 10:00 | 95 | 00:15 | 95 | 01:00 |
| | | | | 54 | 01:00 | 55 | 00:30 |
| | | | | | | 95 | 00:30 |
| B10, B46, B48 | 40 | 95 | 10:00 | 95 | 00:15 | 95 | 01:00 |
| | | | | 62 | 00:45 | 55 | 00:30 |
| | | | | | | 95 | 00:30 |
| GMLL, PTST, CINT, BCTT, RSBI | 40 | 95 | 10:00 | 95 | 00:15 | 95 | 01:00 |
| | | | | 60 | 01:00 | 55 | 00:30 |
| | | | | | | 95 | 00:30 |
| BCTF | 40 | 95 | 10:00 | 95 | 00:15 | 95 | 01:00 |
| | | | | 55 | 00:30 | 55 | 00:30 |
| | | | | 72 | 01:00 | 95 | 00:30 |

### FIT analysis

FIT analysis was performed at Complexo Universitario de Ourense employing the same sample used in the CRC-specific biomarkers analysis. Stool samples for fecal Haemoglobin determination were analyzed using the OC-Sensor tube collector and the assay was performed using the automated OC-Sensor, which detects gastrointestinal bleeding associated with disorders such as CRC, polyps and diverticulitis (Eiken Chemical Co., Tokyo, Japan) as previously described in Cubiella, J. et al., 2016. The sample buffer in the OC-Sensor sample tubes ensures optimum stability and safe transport of the samples, from collection to analysis. Positive tests were those with a concentration of fecal Hemoglobin equal or higher than 100 ng/mL (20 µg Hb/g of feces; FIT100).

### Statistical analysis

In terms of qualitative analysis, absence of biomarker was considered if the obtained Ct value was not comprised within its dynamic range. All statistical analyses were performed using SPSS 23.0 statistical package (IBM, NYC, USA). Significance levels were established for P values ≤ 0.05.

Data normality was assessed through the Kolmogorov-Smirnov test. The non-parametric Kruskal-Wallis test was used to test differences in variables with more than two categories. Pairwise comparisons of subcategories of these variables were analyzed using a Mann-Whitney test. The Bonferroni correction was used to correct for multiple comparisons. All comparisons using bacterial markers were performed between the relative abundances, which were normalized by the dynamic range of each bacterial marker.

The receiver operating characteristic (ROC) curve analysis was applied to determine the usefulness of each biomarker to distinguish among different colonic neoplasia status. The accuracy of discrimination was measured by the area under the ROC curve (AUC).

Machine learning was used to determine which of the studied variables (sex, age, BMI, smoking, bacterial markers, FIT) in combination were capable of distinguishing subjects with advanced neoplasia lesions from those with normal colonoscopy or non-advanced adenomas. The specific methodology consisted of an initial training iteration on 100 aleatory partitions of the dataset and a further validation of the predictive models generated using 4 different machine learning algorithms (neural network, logistic regression, gradient boosting tree, random forest). RAID-CRC was eventually designed using the combination of four of the bacterial markers analyzed together with FIT results.

### Example 2.- Feces biomarkers in neoplasia progression

The relative abundance of each bacterial marker was determined for each diagnostic (normal colonoscopy, non-advanced adenoma, advanced adenoma, CRC) (Figure 1). Regardless of the colonoscopy diagnosis, three different butyrate producing species (B10, B46 and B48) were the most prevalent biomarkers with relative abundance values of 20.4%, 19.0%, and 20.0%, respectively. GMLL and PTST were significantly more abundant in CRC population than in normal colonoscopy individuals (p=0.006 and p<0.001, respectively) or non-advanced adenoma subjects (p=0.047 and p<0.001, respectively). Although with no significant differences, it could be observed a tendency of B46, being more abundant in CRC patients rather than in subjects with advanced adenomas (p=0.087). Interestingly, EUB abundance was maintained constant regardless of neoplasia status. Comparison among the different CRC stages (0, I, II, III, and IV) did not show significant differences in the abundance of any bacterial marker.

### Example 3.- GMLL, PTST, and BCTF can detect advanced neoplasia lesions

The relative abundance of bacterial markers was compared after grouping subjects as follows: (1) normal colonoscopy, (2) neoplasia (non-advanced adenoma + advanced adenoma + CRC), (3) advanced neoplasia (advanced adenoma + CRC), and (4) CRC (Figure 2). PTST was found to be highly correlated with neoplasia lesions (p<0.001). Regarding the detection of advanced neoplasia lesions, GMLL, PTST, and BCTF were potential biomarkers for their detection (p=0.006, p<0.001, and p=0.030, respectively). In terms of prevalence, these three opportunistic pathogens were found more often in patients with advanced neoplasia (GMLL, 64.9%; PTST, 58.4%; and BCTF, 44.7%) than in healthy subjects (GMLL, 53.5%; PTST, 26.1%; and BCTF, 29.8%).

Our results clearly indicate the existence of a bacterial dysbiosis in patients with CRC. The studied bacterial markers were classified according to gut health related phenotypes: butyrate producers (B10, B46, B48, RSBI), opportunistic pathogens (GMLL, PTST, BCTF), hydrogen and oxygen producers (CINT), and saccharolytic species (BCTT) (Figure 3). Relative abundance of these phenotypes was found to change progressively as progression of the disease status. In particular, between subjects with normal colonoscopies and those with CRC we found a decrease in relative abundance of butyrate producers which were replaced by pathogenic bacteria group, being more abundant in CRC and advanced adenoma individuals than in subjects with normal colonoscopies.

Our results show that high abundances of PTST and BCTF correlate with advanced neoplasia, whereas BCTT abundance is correlated with healthy individuals. BCTT is a commensal bacterium commonly found in the gut microbiota of healthy individuals. Commensal bacteria have been observed to attenuate gut inflammation and to contribute to colonization resistance (Macfarlane & Macfarlane, 2012; Baümler & Sperandio, 2016). Hence, high abundances of BCTT correlate with a good intestinal health.

### Example 4.- Combination of CRC bacterial markers and FIT allows a substantial reduction of false-positive results

One the one hand, when FIT100 (20 µg Hb/g of feces) was used, significant differences were observed between subjects with normal colonoscopies or non-advanced adenomas and advanced neoplasia (p<0.001). A 17.1% (19) of the subjects who showed a normal colonoscopy and a 24.3% (27) of those who had non-advanced adenomas showed FIT positive values. These results led to obtain a sensitivity and specificity of 84.0% and 81.0%, respectively, and positive and negative predictive values of 58.0% and 94.0%, respectively (AUC=0.828, 95.0% CI (0.773-0.883)) for the detection of advanced neoplasia. On the other hand, when FIT50 (10 µg Hb/g of feces) was used, a 21.1% (27) of the subjects who showed normal colonoscopy and a 24.2% (31) of those who had non-advanced adenomas showed a false-positive result. FIT50 let to obtain a sensitivity and specificity of 91.0% and 76.0%, respectively, and positive and negative predictive values of 55.0% and 96.0%, respectively (AUC=0.836, 95.0% CI (0.787-0.886)) for the detection of advanced neoplasia. Sensitivity values for bacterial markers alone were much lower being 39.0% for GMLL (AUC=0.622, 95.0% CI (0.541-0.694)), 53.0% for PTST (AUC=0.710, 95.0% CI (0.628-0.776) and 33.0% for BCTF (AUC=0.571, 95.0% CI (0.499-0.656)), while specificity values were comparable.

FIT results, both FIT100 and FIT50, were combined with the fecal bacterial markers in order to know which offered higher performance in terms of sensitivity and specificity values for the detection of advanced neoplasia lesions. The combination of the bacterial markers and FIT100 (RAID CRC - FIT 100) led to obtain a sensitivity of 76.0% and a specificity of 91.0% (Table 5). Nevertheless, these results were slightly improved by FIT50 (RAID CRC - FIT50) as it showed a 4.0% higher sensitivity (80.0%) with similar specificity values for the detection of advanced neoplasia, which therefore was the cut-off value of choice. Thus, a preferred test is based on the combination of EUB, PTST, BCTF, and BCTT with a fecal hemoglobin concentration equal or higher than 50 ng/µL (10 µg Hb/g of feces). Although BCTT did not show significant differences between subjects with normal colonoscopies or non-advanced adenomas and advanced neoplasia subjects, once in combination with EUB, PTST, and BCTF, it was able to increase specificity of the test.

**Table 5. Diagnostic performance of RAID-CRC (using FIT100 and FIT50), FIT100 and FIT50 of the studied symptomatic population compared to FIT100 of screening population. FIT100 (20 µg hemoglobin/g of feces); FIT50 (10 µg hemoglobin/g of feces); PPV, positive predictive value; NPV, negative predictive value.**

| | | **Sensitivity** (%) | **Specificity** (%) | **PPV** (%) | **NPV** (%) |
|---|---|---|---|---|---|
| **RAID-CRC** | Advanced Adenoma | 50 | 91 | 40 | 94 |
| (using FIT100) | Colorectal cancer | 93 | 87 | 55 | 99 |
| | Advanced neoplasia | 76 | 91 | 72 | 92 |
| **RAID-CRC** | Advanced Adenoma | 59 | 90 | 43 | 95 |
| (using FIT50) | Colorectal cancer | 94 | 85 | 51 | 99 |
| | Advanced neoplasia | 80 | 90 | 70 | 94 |
| **FIT100** | Advanced Adenoma | 62 | 81 | 28 | 95 |
| *(this study)* | Colorectal cancer | 98 | 75 | 42 | 99 |
| | Advanced neoplasia | 84 | 81 | 58 | 94 |
| **FIT50** | Advanced Adenoma | 76 | 76 | 28 | 96 |
| *(this study)* | Colorectal cancer | 100 | 71 | 38 | 100 |
| | Advanced neoplasia | 91 | 76 | 55 | 96 |
| **FIT100** | Advanced Adenoma | 28 | 93 | 13 | 97 |
| (Chiu, et al 2016; Bailey et al, 2016) | Colorectal cancer | 78 | 92 | 2 | 99 |
| | Advanced neoplasia | 30 | 93 | 15 | 97 |

The final test (RAID CRC - FIT50 test) comprises the combination of FIT50 and the quantification levels of the three bacterial markers normalized by eubacteria (PTST/EUB, BCTF/EUB, BCTT/EUB), wherein the quantification levels are expressed as Ct value.

Using ratios allowed data normalization, which is critical to control qPCR-associated variables in order to differentiate true biological changes from experimentally induced variation (Vandesompele et al, 2002). Reduction of the fecal hemoglobin concentration threshold from 100 ng/µL to 50 ng/µL in the RAID CRC - FIT 50 test allowed capturing positive subjects that otherwise would be considered false negative with a cut-off value of 100 ng/µL, at expenses of increasing the false-positive rate. However, this effect was diluted in combination with the bacterial markers, since the RAID CRC - FIT 50 test led to an important reduction of false positive results due to an increase of the specificity for detection of advanced neoplasm with respect to FIT50 and FIT100 (Table 5).

The application of the RAID CRC - FIT50 test to the detection of advanced neoplasia resulted in a decrease in the number of false positive results with respect to FIT100, with a 9.7% of the subjects showing a normal colonoscopy and an 11.7% of subjects having non-advanced adenomas. Altogether, the RAID-CRC - FIT50 test was shown to provide a sensitivity and a specificity of 80.0% and 90.0% (AUC=0.837, 95.0% CI (0.730-0.944)), respectively, and positive and negative predictive values of 70.0% and 94.0%, respectively. More importantly, the false-positive rate was reduced by 50.0%, being 46 subjects the false-positive results for FIT100 and 23 subjects for RAID- CRC test - FIT50 test. Interestingly both the RAID CRC - FIT 50 and RAID CRC - FIT 100 tests achieved a similar sensitivity for CRC and AN as the FIT100 protocol but with a higher specificity and positive predictive value (PPV).

In the present work we developed a new methodology suitable to be used in national CRC screening programs using stool samples. Bacterial signatures used in this work were originally retrieved from mucosa samples. Therefore, their presence in feces is not heavily subjected to the variability caused by diet and some external factors (Conlon & Bird, 2015; Vandeputte et al, 2015) but is a measure of the real abundance in the colonic mucosa. This helps to overcome the enormous background noise present in stools and provides physiological meaningfulness to the biomarkers.

No metadata on body mass index (BMI), smoking or feeding habits were available on our dataset. Although these parameters have been reported to influence the microbiota composition of fecal samples (Davis et al, 2017; Yun et al, 2017; Rogers et al, 2012; Capurso & Lahner, 2017; Baothman et al, 2016), as above-mentioned our biomarkers arise from mucosa samples which are not so dependent on external factors (Watt et al, 2016; Durbán et al, 2011). Biedermann et al reported that smoking withdrawal increases microbial diversity (Biedermann et al, 2013). Other studies observed that chronic alcohol consumption leads to an increase in Proteobacteria and a decrease in Bacteroidetes (Kakiyama et al, 2013; Rao et al, 2004). Regarding BMI, its effect on microbiota is controversial (Turnbaugh et al, 2006; Schwiertz et al, 2010; Santacruz et al, 2009). Since the RAID-CRC test described herein is likely to be used in a screening scenario, where the screened population may be affected by a variety of conditions and habits, a non-stratification strategy is found to be a good way to reproduce with utmost reliability the CRC screening scenario.

Cost-effectiveness is also a critical issue in population-based screening (Sonnenberg et al, 2000; McGrath et al, 2002; Telford et al, 2010). Wong and co-workers made a comparison of FIT and colonoscopy in this scenario, showing that FIT was cost-effective manner in averagerisk screening, whereas colonoscopy was cost-effective among higher-risk subject (Wong, 2015). Therefore, combination of FIT with fecal bacterial markers may be superior in terms of cost-effectiveness, since the use of RAID-CRC would permit to save up to 30% of unnecessary colonoscopies. More specifically, the implementation of RAID-CRC in a CRC screening program would result in a reduction of 33,000 colonoscopies due to false positive results when compared to a screening program based on the FOBT (55,000 vs. 22,000 false positives, respectively) (Garcia et al, 2012). Considering the cost of RAID-CRC comparable to that of FOBT, the estimated savings in follow-up colonoscopies after positive screening results would be 77 million € per 100,000 participants in the screening program (Table 6). In addition, using the CRC biomarkers presented in this work may achieve both in developed and in resourcedeprived regions, where colonoscopy facilities are limited, since RAID-CRC represents a potentially viable, cost-effective tool in a CRC screening scenario.

In conclusion, RAID-CRC is a promising tool for CRC screening because of its non-invasiveness, its low cost and its capacity for lowering the number of false-positive results associated with the use of FOBT (e.g., FIT).

**Table 6. Comparison of costs associated to follow-up colonoscopies among different CRC-screening programs.**

| **Factor** | **CRC-screening FIT-based** (Garcia, M. *et al 2012).* | **CRC-screening with combined gFOBT and microbiota profiling** (Zeller, G. *et al.* 2014) | **CRC-screening with combined FIT and fecal bacterial signature (RAID-CRC FIT-50)** |
|---|---|---|---|
| **N° of colonoscopies due to false positive results (per 100,000 screening participants)** | **47.600** | **24,750*** | **22,000 (this study)** |
| **Costs associated to follow-up colonoscopies after a positive screening result** | **111 M €**** | **70 M €**** | **51 M €***** |

| | | | |
|---|---|---|---|
| *The savings have been calculated assuming that combining FOBT with microbiota profiling can increase screening sensitivity more than a 45% relative to FOBT alone. **Associated costs have been calculated considering that the cost of the test is 25 E. ***Associated costs have been calculated considering that the cost of the test is 10 €. | | | |

### References

1. International Agengy for Research on Cancer. GLOBOCAN 2012: Estimated Cancer Incidence, Mortality and Prevalence Worldwide in 2012. Available at: http://globocan.iarc.fr/Pages/fact_sheets_population.aspx. (Accessed: 7th November 2017)
2. Wilson, M. Microbial Inhabitants of Humans: Their Ecology and Role in Health and Disease. (Cambridge, 2005).
3. Brenner, H., Kloor, M. & Pox, C. P. Colorectal cancer. Lancet 383, 1490-1502 (2014).
4. Zauber, A. G. The Impact of Screening on Colorectal Cancer Mortality and Incidence - Has It Really Made a Difference? Dig. Dis. Sci. 60, 681-691 (2015).
5. Elmunzer, B. J. et al. Effect of Flexible Sigmoidoscopy-Based Screening on Incidence and Mortality of Colorectal Cancer: A Systematic Review and Meta-Analysis of Randomized Controlled Trials. PLOS Med. 9, (2012).
6. Rex, D. K. et al. Colorectal Cancer Screening: Recommendations for Physicians and Patients from the U.S. Multi-Society Task Force on Colorectal Cancer. Am. J. Gastroenterol. (2017). doi:10.1038/ajg.2017.174
7. Quintero, E. et al. Colonoscopy versus Fecal Immunochemical Testing in Colorectal-Cancer Screening. N Engl J Med 366, 697-706 (2012).
8. Young, P. E. & Womeldorph, C. M. Colonoscopy for Colorectal Cancer Screening. J. Cancer 4, 217-226 (2013).
9. Rutter, C. M. et al. Adverse Events after Screening and Follow-up Colonoscopy. Cancer Causes Control 23, 289-296 (2012).
10. Sieg, A., Hachmoeller-Eisenbach, U. & Eisenbach, T. Prospective evaluation of complications in outpatient Gl endoscopy: A survey among German gastroenterologists. Gastrointest. Endosc. 53, 620-627 (2001).
11. Gatto, N. M. et al. Risk of perforation after colonoscopy and sigmoidoscopy: a population-based study. J. Natl. Cancer Inst. 95, 230-6 (2003).
12. Sanford, K. W. & McPherson, R. A. Fecal Occult Blood Testing. Clin. Lab. Med. 29, 523-541 (2009).
13. Stracci, F., Zorzi, M., Grazzini, G. & Efird, J. T. Colorectal cancer screening: tests, strategies, and perspectives. (2014). doi:10.3389/fpubh.2014.00210
14. Brenner, H. & Tao, S. Superior diagnostic performance of faecal immunochemical tests for haemoglobin in a head-to-head comparison with guaiac based faecal occult blood test among 2235 participants of screening colonoscopy. Eur. J. Cancer 49, 3049-3054 (2013).
15. Shapiro, J. A. et al. A Comparison of Fecal Immunochemical and High-Sensitivity Guaiac Tests for Colorectal Cancer Screening. Nat. Publ. Gr. (2017). doi:10.1038/ajg.2017.285
16. Mousavinezhad, M., Majdzadeh, R., Akbari Sari, A., Delavari, A. & Mohtasham, F. The effectiveness of FOBT vs. FIT: A meta-analysis on colorectal cancer screening test. Med. J. Islam. Repub. Iran 30 (2016).
17. Goede, S. L. et al. Harms, benefits and costs of fecal immunochemical testing versus guaiac fecal occult blood testing for colorectal cancer screening. doi:10.1371/journal.pone.0172864 (2017).
18. Borges-Canha, M., Portela-Cidade, J. P., Dinis-Ribeiro, M., Leite-Moreira, A. F. & Pimentel-Nunes, P. Role of colonic microbiota in colorectal carcinogenesis: A systematic review. Rev esp enfeRm dig 107, 659-671 (2015).
19. Mira-Pascual, L. et al. Microbial mucosal colonic shifts associated with the development of colorectal cancer reveal the presence of different bacterial and archaeal biomarkers. J. Gastroenterol. 50, 167-179 (2014).
20. Sobhani, I. et al. Microbial Dysbiosis in Colorectal Cancer (CRC) Patients. PLoS One 6, (2011).
21. Jun, X. et al. Molecular characterization of mucosal adherent bacteria and associations with colorectal adenomas. Gut Microbes 138-147 (2010). doi:10.1080/19490976.2016.1241933
22. Kostic, A. D. et al. Fusobacterium nucleatum Potentiates Intestinal Tumorigenesis and Modulates the Tumor-Immune Microenvironment. Cell Host Microbe 14, 207-215 (2013).
23. Zackular, J. P., Rogers, M. A. M., Ruffin, M. T. & Schloss, P. D. The human gut microbiome as a screening tool for colorectal cancer. Cancer Prev. Res. (Phila). 7, 1112-21 (2014).
24. Joshi, A. D. et al. Meat intake, cooking methods, dietary carcinogens, and colorectal cancer risk: findings from the Colorectal Cancer Family Registry (2015).
25. Dulal, S. & Keku, T. O. Gut Microbiome and Colorectal Adenomas. Cancer J. 20, 225-231 (2014).
26. Zeller, G. et al. Potential of fecal microbiota for early-stage detection of colorectal cancer. Mol. Syst. Biol. 10, 766-766 (2014).
27. Marchesi, J. R. et al. Towards the Human Colorectal Cancer Microbiome. PLoS One 6, e20447 (2011).
28. Mas de Xaxars Rivero, T. Descripció i quantificació de la microbiota intestinal associada al càncer colorectal. (2012).
29. Serra-Pagès, M., Garcia-Gil, J., Mas de Xarxars, T. & Aldeguer, X. PCT/EP2015/054451. Biomarkers for early detection, risk screening and monitoring of colorectal cancer and adenomatous polyps. (2015).
30. Cubiella, J. et al. Development and external validation of a faecal immunochemical test-based prediction model for colorectal cancer detection in symptomatic patients. BMC Med. 14, 128 (2016).
31. Levin, B. et al. Screening and Surveillance for the Early Detection of Colorectal Cancer and Adenomatous Polyps, 2008: A Joint Guideline from the American Cancer Society, the US Multi-Society Task Force on Colorectal Cancer, and the American College of Radiology. CA. Cancer J. Clin. 58, 130-160 (2008).
32. Brenner, H., Altenhofen, L., Stock, C. & Hoffmeister, M. Prevention, Early Detection, and Overdiagnosis of Colorectal Cancer Within 10 Years of Screening Colonoscopy in Germany. Clin. Gastroenterol. Hepatol. 13, 717-723 (2015).
33. Shah, R. et al. Biomarkers for Early Detection of Colorectal Cancer and Polyps: Systematic Review. Cancer Epidemiol. Biomarkers Prev. 23, 1712-1728 (2014).
34. Wang, T. et al. Structural segregation of gut microbiota between colorectal cancer patients and healthy volunteers. ISME J. 6, 320-329 (2011).
35. Garrett, W. S. Cancer and the microbiota. Science (80-. ). 348, 80-86 (2015).
36. Kelly, D. & Mulder, I. E. Microbiome and immunological interactions. Nutr. Rev. (2012). doi:10.1111/j.1753-4887.2012.00498.x
37. Feng, Q. et al. Gut microbiome development along the colorectal adenoma-carcinoma sequence. Nat. Commun. 6, (2015).
38. Gao, Z. et al. Microbiota disbiosis is associated with colorectal cancer. (2015). doi:10.3389/fmicb.2015.00020
39. Zhao, L., Zhang, X., Zuo, T. & Yu, J. The Composition of Colonic Commensal Bacteria According to Anatomical Localization in Colorectal Cancer. Engineering 3, 90-97 (2017).
40. Louis, P., Hold, G. L. & Flint, H. J. The gut microbiota, bacterial metabolites and colorectal cancer. Nat. Rev. Microbiol. 12, 661-672 (2014).
41. L. Sears, C. & S. Garrett, W. Microbes, Microbiota and Colon Cancer. Cell Host Microbe 15, 317-328 (2015).
42. Narayanan, V., Peppelenbosch, M. P. & Konstantinov, S. R. Human fecal microbiome-based biomarkers for colorectal cancer. Cancer Prev. Res. (2014). doi:10.1158/1940-6207.CAPR-14-0273
43. Macfarlane, G. T. & Macfarlane, S. Bacteria, Colonic Fermentation, and Gastrointestinal Health. J. AOAC Int. 95, 50-60 (2012).
44. Baümler, A. J. & Sperandio, V. Interactions between the microbiota and pathogenic bacteria in the gut. Nature 535, 85-93 (2016).
45. Vandesompele, J. et al. Accurate normalization of real-time quantitative RT-PCR data by geometric averaging of multiple internal control genes. Genome Biol. 3, 34-1 (2002).
46. Chiu, H. M. et al. Association between early stage colon neoplasms and false-negative results from the fecal immunochemical test. Clin. Gastroenterol. Hepatol. 11, 832-838 (2013).
47. Song, L.-L. & Li, Y.-M. Current noninvasive tests for colorectal cancer screening: An overview of colorectal cancer screening tests. World J. Gastrointest. Oncol. 8, 793 (2016).
48. Bailey, J. R., Aggarwal, A. & Imperiale, T. F. Colorectal Cancer Screening: Stool DNA and Other Noninvasive Modalities. Gut Liver 10, 204-211 (2016).
49. Conlon, M. A. & Bird, A. R. The Impact of Diet and Lifestyle on Gut Microbiota and Human Health. Nutrients 7, 17-44 (2015).
50. Vandeputte, D. et al. Stool consistency is strongly associated with gut microbiota richness and composition, enterotypes and bacterial growth rates. Gut 65, 57-62 (2015).
51. Davis, S. C., Yadav, J. S., Barrow, S. D. & Robertson, B. K. Gut microbiome diversity influenced more by the Westernized dietary regime than the body mass index as assessed using effect size statistic. Microbiologyopen 6, 1-17 (2017).
52. Yun, Y. et al. Comparative analysis of gut microbiota associated with body mass index in a large Korean cohort. BMC Microbiol. 17, (2017).
53. Rogers, M. A. M. et al. Higher Rates of Clostridium difficile Infection among Smokers. (2012). doi:1 0.1371/journal.pone.0042091
54. Capurso, G. & Lahner, E. The interaction between smoking, alcohol and the gut microbiome. Best Pract. Res. Clin. Gastroenterol. 31, 579-588 (2017).
55. Baothman, O. A., Zamzami, M. A., Taher, I., Abubaker, J. & Abu-Farha, M. The role of Gut Microbiota in the development of obesity and Diabetes. Lipids Health Dis. 15, (2016).
56. Watt, E. et al. Extending colonic mucosal microbiome analysis-assessment of colonic lavage as a proxy for endoscopic colonic biopsies. Microbiome 4, (2016).
57. Durbán, A. et al. Assessing Gut Microbial Diversity from Feces and Rectal Mucosa. Microb. Ecol. 61, 123-133 (2011).
58. Biedermann, L. et al. Smoking Cessation Induces Profound Changes in the Composition of the Intestinal Microbiota in Humans. PLoS One 8, (2013).
59. Kakiyama, G. et al. Modulation of the Fecal Bile Acid Profile by Gut Microbiota in Cirrhosis. J. Hepatol. 58, 949-955 (2013).
60. Rao, R. K., Seth, A. & Sheth, P. Recent Advances in Alcoholic Liver Disease I. Role of intestinal permeability and endotoxemia in alcoholic liver disease. Am. J. Physiol. 286, 881-884 (2004).
61. Turnbaugh, P. J. et al. An obesity-associated gut microbiome with increased capacity for energy harvest. Nature 444, 1027-1031 (2006).
62. Schwiertz, A. et al. Microbiota and SCFA in lean and overweight healthy subjects. Obesity 18, 190-195 (2010).
63. Santacruz, A. et al. Interplay between weight loss and gut microbiota composition in overweight adolescents. Obesity 17, 1906-1915 (2009).
64. Sonnenberg, A., Delcò, F. & Inadomi, J. M. Cost-Effectiveness of Colonoscopy in Screening for Colorectal Cancer. Ann. Intern. Med. 133, 573 (2000).
65. McGrath, J. S., Ponich, T. P. & Gregor, J. C. Screening for colorectal cancer: the cost to find an advanced adenoma. Am. J. Gastroenterol. 97, 2902-2907 (2002).
66. Telford, J. J., Levy, A. R., Sambrook, J. C., Zou, D. & Enns, R. A. The cost-effectiveness of screening for colorectal cancer. Can. Med. Assoc. J. 182, 1307-1313 (2010).
67. Wong, M. C., Ching, J. Y., Chan, V. C. & Sung, J. J. The comparative cost-effectiveness of colorectal cancer screening using faecal immunochemical test vs. colonoscopy. (2015). doi:10.1038/srep13568
68. Garcia, M. et al. False-positive results from colorectal cancer screening in Catalonia (Spain), 2000-2010. J. Med. Screen. 19, 77-82 (2012).
69. Matsuda, K. et al. Bacterial Identification by 16S rRNA Gene PCR-Hybridization as a Supplement to Negative Culture Results. J. Clin. Microbiol. 49, 2031-2034 (2011).

## Claims

1. A method for increasing the specificity or reducing the false positive rate of a fecal occult blood test (FOBT) for the screening, diagnosis and/or monitoring of colorectal advanced neoplasia (AN), advanced adenoma (AA) and/ or colorectal cancer (CRC) in a subject, wherein said method comprises:
a. conducting a FOBT in a feces sample isolated from said subject, wherein the FOBT comprises the determination of presence of occult blood in said feces sample; and
b. quantifying in a feces sample isolated from said subject at least the following bacterial markers:
- *Peptostreptococcus stomatis* (PTST),
- *Bacteroides fragilis* (BCTF), and
- *Bacteroides thetaiotaomicron* (BCTT);
wherein the term colorectal advanced neoplasia (AN) encompasses CRC and advanced adenoma (AA).

2. A method for the screening, diagnosis and/or monitoring of colorectal advanced neoplasia (AN), advanced adenoma (AA) and/ or colorectal cancer (CRC) in a subject, wherein said method comprises:
a. conducting a FOBT in a feces sample isolated from said subject, wherein the FOBT comprises the determination of presence of occult blood in said feces sample; and
b. quantifying in a feces sample isolated from said subject at least the following bacterial markers:
- *Peptostreptococcus stomatis* (PTST),
- *Bacteroides fragilis* (BCTF), and
- *Bacteroides thetaiotaomicron* (BCTT);
wherein the term colorectal advanced neoplasia (AN) encompasses CRC and advanced adenoma (AA).

3. A method for the screening, diagnosis and/or monitoring of AN, AA and/or CRC in a human subject according to claim 2, wherein said method further comprises:
c) calculating a combined score from the bacterial markers levels determined in b); and
d) classifying a FOBT positive subject as having AN, AA and/or CRC according to the combined score obtained in c).

4. The method according to claim 3, wherein said method is a method for the screening, and/or diagnosis of AN, AA and/or CRC;
wherein the combined score calculated in c) is proportional to the amount of PTST and BCTF and inversely proportional to the amount of BCTT;
wherein in step d) said method comprises comparing the combined score in the subject's sample with a reference value; and
wherein an increase of the combined score in the subject's sample with regard to said reference value is indicative of AN, AA and/or CRC; wherein said reference value is obtained from subjects not having AN, AA and/or CRC, or from subjects not having a gastrointestinal disease.

5. A method for the screening and/or diagnosis of AN, AA and/or CRC in a human subject according to claim 2, wherein said method further comprises:
i. comparing the amount of GMLL, PTST, and BCTT in the feces sample with corresponding reference values;
ii. wherein increased amount of GMLL and PTST; and a decreased amount of BCTT in the sample of a FOBT positive subject with regard to the corresponding reference value is indicative of AN, AA and/ or CRC; wherein said reference value is obtained from subjects not having AN, AA and/or CRC, or from subjects not having a gastrointestinal disease.

6. The method according to any of claims 1 to 5, wherein said subject is a subject suspected of having CRC or an asymptomatic subject.

7. The method according to any of claims 1 to 6, wherein said FOBT is a fecal immunochemical test (FIT) quantifying human hemoglobin (hHb).

8. The method according to claim 7, wherein a FIT positive subject has hHb levels equal or above 10 µg hHb/g of feces (FIT50 cut-off) or equal or above 20 µg hHb/g of feces (FIT100 cut-off), preferably wherein a FIT positive subject has hHb levels equal or above 10 µg hHb/g of feces (FIT50 cut-off).

9. The method according to any of claims 1 to 8, wherein said method further comprises quantifying one or more bacterial markers selected from the group consisting of B10 (SEQ ID NO:7), B46 (SEQ ID NO:8), B48 (SEQ ID NO:9), *Gemella morbillorum* (GMLL), *Roseburia intestinalis* (RSBI), *Collinsella intestinalis* (CINT), *Faecalibacterium prausnitzii* and/or any of its phylogroups, and *Escherichia coli* (ECO).

10. The method according to any of claims 1 to 9, wherein determination of the levels of PTST, BCTF and BCTT, and optionally the bacterial markers of claim 9, is conducted by qPCR, preferably wherein the quantification levels are expressed as Ct value.

11. The method according to any of claims 1 to 10, wherein the levels of PTST, BCTF and BCTT, and optionally the levels of the bacterial markers of claim 9, are expressed as relative abundance, preferably with respect to eubacteria (EUB) levels.

12. The method according to any of claims 1 to 11, wherein said method further comprises storing the method results in a data carrier, preferably wherein said data carrier is a computer readable medium.

13. A method is as defined in any of claims 3 to 12, wherein the combined score of step c) is calculated using a computer; and/or the selection, classification and/or determination of step d) is conducted using a computer.

14. A kit suitable for quantifying PTST, BCTF and BCTT, wherein said kit comprises the following reagents:
- an oligonucleotide comprising or consisting of any of SEQ ID NO:24, or SEQ ID NO:25, or a sequence with at least 80% identity to any thereto;
- an oligonucleotide comprising or consisting of any of SEQ ID NO:26, or SEQ ID NO:27, or a sequence with at least 80% identity to any thereto; and
- an oligonucleotide comprising or consisting of any of SEQ ID NO:30, or SEQ ID NO:31, or a sequence with at least 80% identity to any thereto; and
- optionally, an oligonucleotide suitable for the quantification of Eubacteria, preferably an oligonucleotide comprising or consisting of any of SEQ ID NO: 5, SEQ ID NO: 6, SEQ ID NO:14, or SEQ ID NO:15, or a sequence with at least 80% identity to any thereto;
- optionally, further comprising instructions for the use of said reagents in determining said bacterial markers levels in a feces sample isolated from a subject.

15. Use of a kit according to claim 14, in a method for the screening, diagnosis and/or monitoring of AN, AA and/or CRC according to any of claims 1 to 13.

## Patentansprüche

1. Verfahren zum Erhöhen der Spezifizität oder zum Verringern der Falsch-positiv-Rate eines fäkalen okkulten Bluttests (FOBT) zum Screening, Diagnostizieren und/oder Überwachen von kolorektaler fortgeschrittener Neoplasie (AN), fortgeschrittenem Adenom (AA) und/oder Kolorektalkrebs (CRC) in einem Individuum, wobei das genannte Verfahren Folgendes umfasst:
a. das Durchführen eines FOBT in einer Stuhlprobe isoliert aus dem genannten Individuum, wobei der FOBT die Bestimmung der Anwesenheit von okkultem Blut in der genannten Stuhlprobe umfasst; und
b. das Quantifizieren in einer Stuhlprobe isoliert aus dem genannten Individuum mindestens der folgenden bakteriellen Marker:
- *Peptostreptococcus stomatis* (PTST),
- *Bacteroides fragilis* (BCTF) und
- *Bacteroides thetaiotaomicron* (BCTT);
wobei der Begriff kolorektale fortgeschrittene Neoplasie (AN) CRC und fortgeschrittenes Adenom (AA) umfasst.

2. Verfahren zum Screening, Diagnostizieren und/oder Überwachen von kolorektaler fortgeschrittener Neoplasie (AN), fortgeschrittenem Adenom (AA) und/oder Kolorektalkrebs (CRC) in einem Individuum, wobei das genannte Verfahren Folgendes umfasst:
a. das Durchführen eines FOBT in einer Stuhlprobe isoliert aus dem genannten Individuum, wobei der FOBT die Bestimmung der Anwesenheit von okkultem Blut in der genannten Stuhlprobe umfasst; und
b. das Quantifizieren in einer Stuhlprobe isoliert aus dem genannten Individuum mindestens der folgenden bakteriellen Marker:
- *Peptostreptococcus stomatis* (PTST),
- *Bacteroides fragilis* (BCTF) und
- *Bacteroides thetaiotaomicron* (BCTT);
wobei der Begriff kolorektale fortgeschrittene Neoplasie (AN) CRC und fortgeschrittenes Adenom (AA) umfasst.

3. Verfahren zum Screening, Diagnostizieren und/oder Überwachen von AN, AA und/oder CRC in einem menschlichen Individuum nach Anspruch 2, wobei das genannte Verfahren zusätzlich Folgendes umfasst:
c) das Berechnen einer kombinierten Punktzahl aus den in b) bestimmten bakteriellen Markerniveaus; und
d) das Klassifizieren eines FOBT-positiven Individuums als AN, AA und/oder CRC aufweisend gemäß der in c) erhaltenen kombinierten Punktzahl.

4. Verfahren nach Anspruch 3, wobei das genannte Verfahren ein Verfahren zum Screening und/oder Diagnostizieren von AN, AA und/oder CRC ist;
wobei die in c) berechnete kombinierte Punktzahl proportional zur Menge von PTST und BCTF ist und umgekehrt proportional zur Menge von BCTT ist;
wobei in Schritt d) das genannte Verfahren das Vergleichen der kombinierten Punktzahl in der Probe des Individuums mit einem Referenzwert umfasst; und
wobei eine Erhöhung der kombinierten Punktzahl in der Probe des Individuums in Bezug auf den genannten Referenzwert auf AN, AA und/oder CRC schließen lässt; wobei der genannte Referenzwert aus Individuen, welche nicht AN, AA und/oder CRC aufweisen, oder aus Individuen, welche keine Magen-Darm-Erkrankung aufweisen, erhalten wird.

5. Verfahren zum Screening und/oder Diagnostizieren von AN, AA und/oder CRC in einem menschlichen Individuum nach Anspruch 2, wobei das genannte Verfahren zusätzlich Folgendes umfasst:
i. das Vergleichen der Menge von GMLL, PTST und BCTT in der Stuhlprobe mit entsprechenden Referenzwerten;
ii. wobei eine erhöhte Menge von GMLL und PTST; und eine verringerte Menge von BCTT in der Probe eines FOBT-positiven Individuums in Bezug auf den entsprechenden Referenzwert auf AN, AA und/oder CRC schließen lässt; wobei der genannte Referenzwert aus Individuen, welche nicht AN, AA und/oder CRC aufweisen, oder aus Individuen, welche keine Magen-Darm-Erkrankung aufweisen, erhalten wird.

6. Verfahren nach einem der Ansprüche 1 bis 5, wobei das genannte Individuum ein Individuum, von welchem es vermutet wird, CRC aufzuweisen, oder ein asymptomatisches Individuum ist.

7. Verfahren nach einem der Ansprüche 1 bis 6, wobei der genannte FOBT ein immunchemischer Stuhltest (FIT) ist, welcher menschliches Hämoglobin (hHb) quantifiziert.

8. Verfahren nach Anspruch 7, wobei ein FIT-positives Individuum hHb-Niveaus gleich oder höher als 10 µg hHb/g Stuhl (FIT50-Grenze) oder gleich oder höher als 20 µg hHb/g Stuhl (FIT100-Grenze) aufweist, wobei vorzugsweise ein FIT-positives Individuum hHb-Niveaus gleich oder höher als 10 µg hHb/g Stuhl (FIT50-Grenze) aufweist.

9. Verfahren nach einem der Ansprüche 1 bis 8, wobei das genannte Verfahren zusätzlich das Quantifizieren eines oder mehrerer bakteriellen Marker ausgewählt aus der Gruppe bestehend aus B10 (SEQ ID NO:7), B46 (SEQ ID NO:8), B48 (SEQ ID NO:9), *Gemella morbillorum* (GMLL), *Roseburia intestinalis* (RSBI), *Collinsella intestinalis* (CINT), *Faecalibacterium prausnitzii* und/oder einer dessen Phylogruppen, und *Escherichia coli* (ECO) umfasst.

10. Verfahren nach einem der Ansprüche 1 bis 9, wobei die Bestimmung der Niveaus von PTST, BCTF und BCTT, und wahlweise der bakteriellen Marker nach Anspruch 9, mittels qPCR durchgeführt, wobei vorzugsweise die Quantifizierungsniveaus als Ct-Wert ausgedrückt werden.

11. Verfahren nach einem der Ansprüche 1 bis 10, wobei die Niveaus von PTST, BCTF und BCTT, und wahlweise die Niveaus der bakteriellen Marker nach Anspruch 9, als relative Häufigkeit, vorzugsweise in Bezug auf die Eubakterien (EUB)-Niveaus, ausgedrückt werden.

12. Verfahren nach einem der Ansprüche 1 bis 11, wobei das genannte Verfahren zusätzlich das Speichern der Verfahrensergebnisse in einem Datenträger umfasst, wobei vorzugsweise der genannte Datenträger ein computerlesbares Medium ist.

13. Verfahren nach einem der Ansprüche 3 bis 12, wobei die kombinierte Punktzahl aus Schritt c) unter Verwendung eines Computers berechnet wird; und/oder die Auswahl, die Klassifizierung und/oder die Bestimmung aus Schritt d) unter Verwendung eines Computers durchgeführt wird.

14. Kit geeignet für die Quantifizierung von PTST, BCTF und BCTT, wobei das genannte Kit die folgenden Reagenzien umfasst:
- ein Oligonukleotid umfassend oder bestehend aus einer der SEQ ID NO:24 oder SEQ ID NO:25, oder einer Sequenz mit mindestens 80 % Identität mit einer derselben;
- ein Oligonukleotid umfassend oder bestehend aus einer der SEQ ID NO:26 oder SEQ ID NO:27, oder einer Sequenz mit mindestens 80 % Identität mit einer derselben; und
- ein Oligonukleotid umfassend oder bestehend aus einer der SEQ ID NO:30 oder SEQ ID NO:31, oder einer Sequenz mit mindestens 80 % Identität mit einer derselben; und
- wahlweise, ein Oligonukleotid geeignet für die Quantifizierung von Eubakterien, vorzugsweise ein Oligonukleotid umfassend oder bestehend aus einer der SEQ ID NO: 5, SEQ ID NO: 6, SEQ ID NO:14 oder SEQ ID NO:15, oder einer Sequenz mit mindestens 80 % Identität mit einer derselben;
- wahlweise, zusätzlich umfassend Anleitungen für die Verwendung der genannten Reagenzien bei der Bestimmung der genannten bakteriellen Markerniveaus in einer Stuhlprobe isoliert aus einem Individuum.

15. Verwendung eines Kits nach Anspruch 14, in einem Verfahren zum Screening, Diagnostizieren und/oder Überwachen von AN, AA und/oder CRC nach einem der Ansprüche 1 bis 13.

## Revendications

1. Procédé pour augmenter la spécificité ou réduire le taux de faux positifs d'un test de sang occulte dans les selles (FOBT) pour le criblage, le diagnostic et/ou la surveillance d'une néoplasie colorectale avancée (AN), d'un adénome avancé (AA) et/ou d'un cancer colorectal (CRC) chez un sujet, dans lequel ledit procédé comprend :
a. réaliser un FOBT dans un échantillon de selles isolé à partir dudit sujet, dans lequel le FOBT comprend la détermination de la présence de sang occulte dans ledit échantillon de selles ; et
b. quantifier dans un échantillon de selles isolé à partir dudit sujet au moins les marqueurs bactériens suivants :
- *Peptostreptococcus stomatis* (PTST),
- *Bacteroides fragilis* (BCTF), et
- *Bacteroides thetaiotaomicron* (BCTT) ;
dans lequel le terme néoplasie colorectale avancée (AN) englobe le CRC et l'adénome avancé (AA).

2. Procédé pour le criblage, le diagnostic et/ou la surveillance d'une néoplasie colorectale avancée (AN), d'un adénome avancé (AA) et/ou d'un cancer colorectal (CRC) chez un sujet, dans lequel ledit procédé comprend :
a. réaliser un FOBT dans un échantillon de selles isolé à partir dudit sujet, dans lequel le FOBT comprend la détermination de la présence de sang occulte dans ledit échantillon de selles ; et
b. quantifier dans un échantillon de selles isolé à partir dudit sujet au moins les marqueurs bactériens suivants :
- *Peptostreptococcus stomatis* (PTST),
- *Bacteroides fragilis* (BCTF), et
- *Bacteroides thetaiotaomicron* (BCTT) ;
dans lequel le terme néoplasie colorectale avancée (AN) englobe le CRC et l'adénome avancé (AA).

3. Procédé pour le criblage, le diagnostic et/ou la surveillance d'une AN, d'un AA et/ou d'un CRC chez un sujet humain selon la revendication 2, dans lequel ledit procédé comprend en outre :
c) calculer un score combiné à partir des niveaux de marqueurs bactériens déterminés en b) ; et
d) classer un sujet positif au FOBT comme souffrant une AN, un AA et/ou un CRC en fonction du score combiné obtenu en c).

4. Procédé selon la revendication 3, dans lequel ledit procédé est un procédé pour le criblage, et/ou le diagnostic d'une AN, d'un AA et/ou d'un CRC ;
dans lequel le score combiné calculé en c) est proportionnel à la quantité de PTST et BCTF et inversement proportionnel à la quantité de BCTT ;
dans lequel, dans l'étape d), ledit procédé comprend comparer le score combiné dans l'échantillon du sujet avec une valeur de référence ; et
dans lequel une augmentation du score combiné dans l'échantillon du sujet par rapport audit valeur de référence est indicative d'une AN, d'un AA et/ou d'un CRC ; dans lequel ladite valeur de référence est obtenue à partir de sujets ne souffrant pas d'une AN, d'un AA et/ou d'un CRC, ou à partir de sujets ne souffrant pas d'une maladie gastrointestinale.

5. Procédé pour le criblage et/ou le diagnostic d'une AN, d'un AA et/ou d'un CRC chez un sujet humain selon la revendication 2, dans lequel ledit procédé comprend en outre :
i. comparer la quantité de GMLL, PTST, et BCTT dans l'échantillon de selles avec des valeurs de référence correspondantes ;
ii. dans lequel une quantité augmentée de GMLL et de PTST ; et une quantité réduite de BCTI dans l'échantillon d'un sujet positif au FOBT par rapport à la valeur de référence correspondante est indicative d'une AN, d'un AA et/ou d'un CRC ; dans lequel ladite valeur de référence est obtenue à partir de sujets ne souffrant pas d'une AN, d'un AA et/ou d'un CRC, ou à partir de sujets ne souffrant pas d'une maladie gastrointestinale.

6. Procédé selon l'une quelconque des revendications 1 à 5, dans lequel ledit sujet est un sujet suspecté de souffrir d'un CRC ou un sujet asymptomatique.

7. Procédé selon l'une quelconque des revendications 1 à 6, dans lequel ledit FOBT est un test immunochimique fécal (FIT) quantifiant l'hémoglobine humaine (hHb).

8. Procédé selon la revendication 7, dans lequel un sujet positif au FIT présente des niveaux de hHb égaux ou supérieurs à 10 µg de hHb/g de selles (seuil de coupure de FIT50) ou égaux ou supérieurs à 20 µg de hHb/g de selles (seuil de coupure de FIT100), de préférence dans lequel un sujet positif au FIT présente des niveaux de hHb égaux ou supérieurs à 10 µg de hHb/g de selles (seuil de coupure de FIT50).

9. Procédé selon l'une quelconque des revendications 1 à 8, dans lequel ledit procédé comprend en outre quantifier un ou plusieurs marqueurs bactériens choisis parmi le groupe constitué par B10 (SEQ ID NO : 7), B46 (SEQ ID NO : 8), B48 (SEQ ID NO: 9), *Gemella morbillorum* (GMLL), *Roseburia intestinalis* (RSBI), *Collinsella intestinalis* (CINT), *Faecalibacterium prausnitzii* et/ou l'un quelconque de ces phylogroupes, et *Escherichia coli* (ECO).

10. Procédé selon l'une quelconque des revendications 1 à 9, dans lequel la détermination des niveaux de PTST, de BCTF et de BCTT, et optionnellement les marqueurs bactériens selon la revendication 9, est réalisée par qPCR, de préférence dans lequel les niveaux de quantification sont exprimés comme valeur de Ct.

11. Procédé selon l'une quelconque des revendications 1 à 10, dans lequel les niveaux de PTST, de BCTF et de BCTT, et optionnellement les niveaux des marqueurs bactériens selon la revendication 9, sont exprimés comme abondance relative, de préférence par rapport aux niveaux d'eubactéries (EUB).

12. Procédé selon l'une quelconque des revendications 1 à 11, dans lequel ledit procédé comprend en outre stocker les résultats de procédé dans un support de données, de préférence dans lequel ledit support de données est un support lisible par ordinateur.

13. Procédé selon l'une quelconque des revendications 3 à 12, dans lequel le score combiné de l'étape c) est calculé en utilisant un ordinateur ; et/ou la sélection, classification et/ou détermination de l'étape d) est réalisée en utilisant un ordinateur.

14. Kit approprié pour quantifier PTST, BCTF et BCTT, dans lequel ledit kit comprend les réactifs suivants :
- un oligonucléotide comprenant ou constitué par l'une quelconque de SEQ ID NO : 24, ou SEQ ID NO : 25, ou une séquence ayant une identité d'au moins 80 % par rapport à l'une quelconque de celles-ci ;
- un oligonucléotide comprenant ou constitué par l'une quelconque de SEQ ID NO : 26, ou SEQ ID NO : 27, ou une séquence ayant une identité d'au moins 80 % par rapport à l'une quelconque de celles-ci ; et
- un oligonucléotide comprenant ou constitué par l'une quelconque de SEQ ID NO : 30, ou SEQ ID NO : 31, ou une séquence ayant une identité d'au moins 80 % par rapport à l'une quelconque de celles-ci ; et
- optionnellement, un oligonucléotide approprié pour la quantification d'eubactéries, de préférence un oligonucléotide comprenant ou constitué par l'une quelconque de SEQ ID NO : 5, SEQ ID NO : 6, SEQ ID NO : 14, ou SEQ ID NO : 15, ou une séquence ayant une identité d'au moins 80 % par rapport à l'une quelconque de celles-ci ;
- optionnellement, comprenant en outre des instructions pour l'utilisation desdits réactifs dans la détermination desdits niveaux de marqueurs bactériens dans un échantillon de selles isolé à partir d'un sujet.

15. Utilisation d'un kit selon la revendication 14, dans un procédé pour le criblage, le diagnostic et/ou la surveillance d'une AN, d'un AA et/ou d'un CRC selon l'une quelconque des revendications 1 à 13.
